# EUROPEAN PATENT APPLICATION

(11) **EP 1 710 249 A1**
(43) Date of publication of application: **11.10.2006**
(21) Application number: 05704111.3
(22) Date of filing: 26.01.2005
(51) Int. Cl.: C07H 19/10, C07H 19/20, C07H 21/02, C07H 21/04, A61K 31/7088, A61K 31/7068, A61K 31/7072, A61K 31/7076

(54) **RIBONUCLEIC ACID COMPOUND AND METHOD OF LIQUID-PHASE SYNTHESIS OF OLIGONUCLEIC ACID COMPOUND**

(30) Priority: 27.01.2004 JP 2004018060
(71) Applicant: Nippon Shinyaku Co., Ltd., Kyoto-shi, Kyoto 601-8550 (JP)
(72) Inventor: OHGI, Tadaaki, Tsuchiura-shi, Ibaraki 3000013 (JP); UEDA, Toshihiro, Tsukuba-shi, Ibaraki 3050003 (JP); MARUYAMA, Yasufumi, Kyoto-shi, Kyoto 615-8118 (JP); MASUDA, Hirofumi, Yuki-gun, Ibaraki 3002742 (JP)
(74) Representative: Vossius & Partner
(86) International application number: PCT/JP2005/000974
(87) International publication number: WO 2005/070946

(57) **Abstract**

A novel phosphotriesterified ribonucleic acid compound which is important for liquid-phase synthesis of oligo-RNA is provided.

Examples of the ribonucleic acid compound of the invention may include ribonucleic acid compounds represented by the following general formula: 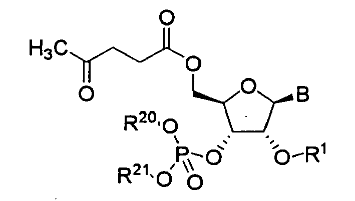 (wherein B represents adenine, guanine, cytosine or uracil or a modified form thereof; R²¹ represents aryl which may be substituted or a monocyclic or bicyclic heterocyclic group which may be substituted; R²⁰ represents H or alkyl which may be substituted; and R¹ represents a protecting group which can be removed at 90% or more at a temperature in the range from 0°C to 60°C under acidic conditions at a pH value from 2 to 4 within 24 hours).

## Description

### Technical Field

The present invention relates to a novel phosphotriesterified ribonucleic acid compound which is important for liquid-phase synthesis of oligo-RNA useful as a therapeutic agent, a diagnostic agent, or a reagent for research.

### Background Art

At present, oligonucleotide compounds play an important role in therapeutic or diagnostic method. In general, three methods are known as methods for producing oligonucleotide compounds, that is, the phosphotriester method developed by Reese (Tetrahedron 1978, 34, 3143), the amidite method developed by Beaucage (Methods in Molecular Biology: Protocols for Oligonucleotides and Analogs; Agrawal, ed.; Humana Press: Totowa, 1993, Vol. 20. 33-61) and the H-phosphonate method developed by Froehler (Methods in Molecular Biology: Protocols for Oligonucleotides and Analogs; Agrawal, ed.; Humana Press: Totowa, 1993, Vol. 20, 63-80). The phosphotriester method is mainly used in liquid-phase synthesis, and the amidite method and the H-phosphonate method are mainly used in solid-phase synthesis.
At present, production methods used in production of oligonucleotide compounds are solid-phase methods. The solid-phase methods can easily achieve oligomer production, however, because the reaction is carried out on a resin, the reaction site is limited, and it is difficult to scale up.
In order to supply a large amount of oligonucleotide compound, it is essential to develop a method for synthesizing an oligonucleotide compound in a liquid-phase. However, at present, it is only oligo-DNA that can be supplied in a large amount among oligonucleotide compounds, and a liquid-phase synthesis method suitable for producing a large amount of oligo-RNA has not been known.

In order to perform a liquid-phase synthesis of oligo-RNA, it is necessary that a large amount of highly protected ribonucleic acid compound which is a monomer component that can be used as a raw material in the phosphotriester method and in which the hydroxyl at the 3'-position of ribose is a phosphotriester form and all the protecting groups can be easily deprotected in a final stage of stepwise oligomerization can be supplied.
As requirements for protecting groups for each functional group of the highly protected ribonucleic acid compound, which is a monomer component necessary for oligo-RNA synthesis, the following requirements are conceivable. (1) Oligo-RNA can be stably present under condensation reaction conditions. (2) When another protecting group is present in a molecule, the respective protecting groups can be deprotected selectively under different conditions. (3) Under conditions in which oligo-RNA can be stably present, only a protecting group can be easily deprotected.

Examples of the protecting group for the hydroxyl at the 2'-position of ribose may include silicon protecting groups such as t-butyldimethylsilyl, acetal protecting groups such as a 2-tetrahydrofuranyl group and a 2-tetrahydropyranyl group. In particular, such an acetal protecting group can be removed under mild aqueous acidic conditions at a pH value from 2 to 4, in which a side reaction such as phosphotransfer or fragmentation accompanying phosphotransfer does not occur in a deprotection step for oligo-RNA.
Examples of the protecting group for the hydroxyl at the 5'-position of ribose may include 4,4'-dimethoxytrityl and levulinyl. In the case where an acetal protecting group is used as the protecting group for the hydroxyl at the 2'-position of ribose, when a 4,4'-dimethoxytrityl group is used as the protecting group for the hydroxyl at the 5'-position of ribose in the final condensation step for producing oligo-RNA, both the protecting groups can be removed under acidic conditions in the final deprotection step. However, when the 4,4'-dimethoxytrityl group is used as the protecting group for the hydroxyl at the 5'-position of ribose in the process of producing oligo-RNA, it is difficult to allow the acetal protecting group to exist as it is, therefore, it is better to avoid using the 4,4'-dimethoxytrityl group.
When levulinyl, CH₃-CO-(CH₂)₂-CO-, is used as the protecting group for the hydroxyl at the 5'-position of ribose instead, it can be deprotected by a treatment with hydrazine under neutral conditions in which the acetal protecting group is not removed.

As a compound in which these two types of protecting groups, levulinyl and tetrahydrofuranyl, are combined, a ribonucleic acid compound in which the hydroxyl at the 3'-position of ribose has been amidated, and which can be used in a solid-phase synthesis method for oligo-RNA is known (see, for example, Non-patent document 1).
However, a ribonucleic acid compound in which the hydroxyl at the 2'-position of ribose is protected with acetal, the hydroxyl at the 5'-position of ribose has been levulinylated and the hydroxyl at the 3'-position of ribose has been phosphotriesterified, and which can be used in a liquid-phase synthesis method for oligo-RNA, has not been known yet.

Conventionally, it is known that a compound in which levulinyl has been introduced into the hydroxyl at the 5'-position of ribose can be produced using a chemical method. For example, a method in which levulinic acid and 2-chloro-1-methylpyridinium iodide are reacted with a ribonucleic acid compound in which the 2'-position of ribose is protected with tetrahydrofuranyl and hydroxyl is located at the 3'- and 5'-positions of ribose, is known (see, for example Non-patent document 1). However, with this method, only the hydroxyl at the 5'-position of ribose cannot be selectively levulinylated, and only the hydroxyl at the 3'-position of ribose is levulinylated, or both the hydroxyl groups at the 3'- and 5'-positions of ribose are levulinylated. In order to separate these, a purification step or other steps become necessary, and an objective compound in which only the hydroxyl at the 5'-position of ribose has been levulinylated is obtained with a low yield (30% to 68%). In order to obtain a large amount of raw material, considerable time and cost are needed.

On the other hand, although it has nothing to do with the synthesis of oligo-RNA, a method in which acylation is performed using a lipase for.a ribonucleic acid compound, in which hydroxyl is located at the 3'- and 5'-positions of ribose, is known (see, for example, Non-patent document 2). As the lipase, Candida antarctica lipase B (CAL-B) is used, and the hydroxyl at the 5'-position of ribose of a nucleic acid compound is regioselectively acylated. In addition, although it also has nothing to do with the synthesis of oligo-RNA, a method in which by using a similar technique, the hydroxyl at the 5'-position of ribose is regioselectively levulinylated for a nucleic acid compound in which DNA or the hydroxyl at the 2'-position of ribose has been substituted with a substituent, such as methoxyethoxy and methyl, which is not easily removed, is known (see, for example, Patent document 1).
However, a method in which regioselective levulinylation is performed using a lipase for a ribonucleic acid compound that can be deprotected under conditions in which the compound can be stably present, which is essential for oligo-RNA synthesis, for example, a ribonucleic acid compound having a protecting group that can be removed at 90% or more at a temperature in the range from 0°C to 60°C under acidic conditions at a pH value from 2 to 4 within 24 hours at the hydroxyl at the 2'-position of ribose has not been known.
Non-patent document 1: Tetrahedron Vol. 46, No. 19, pp. 6673-6688, 1990
Non-patent document 2: Journal of Organic Chemistry Vol. 58, No. 3, pp. 653-660, 1993
Patent document 1: WO 02/079215

### Disclosure of the Invention

### Problems that the Invention is to Solve

A main object of the present invention is to provide a novel phosphotriesterified ribonucleic acid compound which is important for liquid-phase synthesis of oligo-RNA.
Further, another object of the present invention is to provide a method of regioselectively levulinylating the hydroxyl at the 5'-position of ribose for a ribonucleic acid compound in which the hydroxyl at the 2'-position of ribose is protected with a protecting group that can be removed at 90% or more at a temperature in the range from 0°C to 60°C under acidic conditions at a pH value from 2 to 4 within 24 hours, which is important for producing a phosphotriesterified ribonucleic acid compound.
Further, another object of the present invention is to provide a novel liquid-phase synthesis method for oligo-RNA.

### Means for Solving the Problems

As a result of extensive studies, the present inventors have found that the hydroxyl at the 5'-position of ribose using a lipase, the above objects can be achieved by way of levulinylating the hydroxyl at the 5'-position of ribose using a lipase, and thus the present invention has been completed.
In the present invention, a ribonucleic acid compound represented by the following general formula (1) (hereinafter referred to as "the compound of the present invention") can be included. 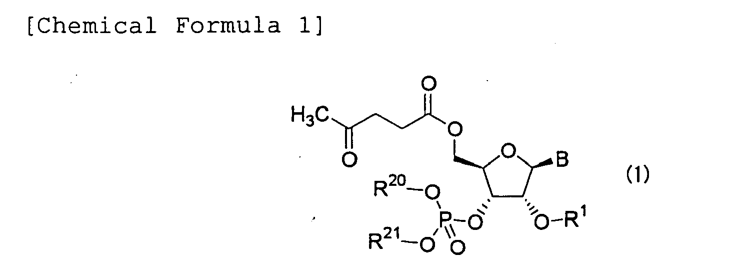 In the formula, B represents adenine, guanine, cytosine or uracil or a modified form thereof. R²⁰ represents H or alkyl which may be substituted. R²¹ represents aryl which may be substituted or a monocyclic or bicyclic heterocyclic group which may be substituted. R¹ represents a protecting group which can be removed at 90% or more at a temperature in the range from 0°C to 60°C under acidic conditions at a pH value from 2 to 4 within 24 hours. Preferably, it is a protecting group which can be removed at 90% or more at a temperature in the range from 15°C to 40°C under acidic conditions at a pH value from 2 to 4 within 24 hours. More preferably, it is 2-tetrahydrofuranyl or 1,3-dioxolan-2-yl.

Examples of the "alkyl" related to R²⁰ may include straight-chain or branched-chain alkyl having 1 to 4 carbon atoms such as methyl, ethyl, n-propyl, isopropyl, n-butyl, isobutyl, sec-butyl, and tert-butyl. The "alkyl" related to R²⁰ may be substituted, and the alkyl is substituted by 1 to 3 same or different substituents which can be selected from the group consisting of halogen, alkyl, alkoxy, cyano and nitro. Examples of the "halogen" which is a substituent for the "alkyl" may include fluorine, chlorine, bromine and iodine. Examples of the "alkyl" which is a substituent for the "alkyl" may include straight-chain or branched-chain alkyl having 1 to 4 carbon atoms such as methyl, ethyl, n-propyl, isopropyl, n-butyl, isobutyl, sec-butyl, and tert-butyl. Examples of the "alkoxy" which is a substituent for the "alkyl" may include straight-chain or branched-chain alkoxy having 1 to 4 carbon atoms such as methoxy, ethoxy, n-propoxy, an isopropoxy, n-butoxy, isobutoxy, a sec-butoxy and a tert-butoxy. In particular, R²⁰ is preferably 2-cyanoethyl, 2,2,2-trichloroethyl or 2,2,2-tribromoethyl.

Examples of the "aryl" related to R²¹ may include aryl having 6 to 12 carbon atoms such as phenyl, 1-naphthyl, 2-naphthyl and biphenyl. The "aryl" may be substituted, and the aryl is substituted by 1 to 3 same or different substituents which can be selected from the group consisting of halogen, alkyl, alkoxy , cyano and nitro. Examples of the "halogen" which is a substituent for the "aryl" may include fluorine, chlorine, bromine and iodine. Examples of the "alkyl" which is a substituent for the "aryl" may include straight-chain or branched-chain alkyl having 1 to 4 carbon atoms such as methyl, ethyl, n-propyl , isopropyl, an n-butyl, isobutyl , sec-butyl, and tert-butyl. Examples of the "alkoxy" which is a substituent for the "aryl" may include straight-chain or branched-chain alkoxy having 1 to 4 carbon atoms such as methoxy, ethoxy, n-propoxy, isopropoxy, n-butoxy, isobutoxy, sec-butoxy and tert-butoxy. In particular, 2-chlorophenyl and 2-chloro-4-tert-butylphenyl are preferred.

Examples of the "monocyclic or bicyclic heterocyclic group which may be substituted" related to R²¹ may include 5- to 12-membered monocyclic or condensed rings which contain 1 to 3 heteroatoms selected from the group consisting of a nitrogen atom, an oxygen atom and a sulfur atom and may have 1 to 6 unsaturated bonds. As the substituent, for example, the same or different 1 to 3 substituents selected from the group consisting of alkyl , alkoxy, halogen and nitro can be exemplified. Examples of the "halogen" which is a substituent for the "monocyclic or bicyclic heterocyclic group which may be substituted" may include fluorine, chlorine, bromine and iodine. Examples of the "alkyl " which is a substituent for the "monocyclic or bicyclic heterocyclic group which may be substituted" may include straight-chain or branched-chain alkyl having 1 to 4 carbon atoms such as methyl , ethyl, n-propyl, isopropyl, n-butyl, isobutyl, sec-butyl and tert-butyl. Examples of the "alkoxy " which is a substituent for the "monocyclic or bicyclic heterocyclic group which may be substituted" may include straight-chain or branched-chain alkoxy having 1 to 4 carbon atoms such as methoxy, ethoxy, n-propoxy, isopropoxy, n-butoxy, isobutoxy, sec-butoxy and tert-butoxy. In particular, R²¹ is preferably 1-benzotriazole or 1-morpholino.

The "modified form" as used herein refers to a compound in which a heterocyclic base moiety has been substituted with an arbitrary substituent.
As the substituent related to the "modified form" of B, the same or different 1 to 3 substituents selected from the group consisting of halogen, acyl, alkyl, arylalkyl, alkoxy, alkoxyalkyl, hydroxyl, amino, a monoalkylamino group, a dialkylamino group, carboxyl, cyano and nitro can be exemplified.
Examples of the "alkoxy" related to the modified form of B may include straight-chain or branched-chain alkoxy having 1 to 4 carbon atoms such as methoxy, ethoxy, n-propoxy, isopropoxy, n-butoxy, isobutoxy, sec-butoxy and tert-butoxy. Among these, those having 1 to 4 carbon atoms are preferred, and particularly, methoxy is preferred. Examples of the alkoxy moiety of the "alkoxyalkyl" also may include the same ones as illustrated in the explanation of the "alkoxy" related to the modified form of B.
Examples of the "halogen" related to the modified form of B may include fluorine, chlorine, bromine and iodine.

Examples of the "alkyl" related to the modified form of B may include straight-chain or branched-chain alkyl having 1 to 4 carbon atoms such as methyl, ethyl, n-propyl, isopropyl, n-butyl, isobutyl, sec-butyl, and tert-butyl. Examples of the alkyl moiety of the "arylalkyl", "alkoxyalkyl", "monoalkylamino group" and "dialkylamino group" also may include the same ones as illustrated in the explanation of the "alkyl" related to the modified form of B. The "alkyl" related to the modified form of B may be substituted, and the alkyl is substituted by 1 to 3 same or different substituents which can be selected from the group consisting of halogen, alkyl, alkoxy, cyano and nitro. Examples of the "halogen" which is a substituent for the "alkyl" may include fluorine, chlorine, bromine and iodine. Examples of the "alkyl" which is a substituent for the "alkyl" may include straight-chain or branched-chain alkyl having 1 to 4 carbon atoms such as methyl, ethyl, n-propyl, isopropyl, n-butyl, isobutyl, sec-butyl, and tert-butyl. Examples of the "alkoxy" which is a substituent for the "alkyl" may include straight-chain or branched-chain alkoxy having 1 to 4 carbon atoms such as methoxy, ethoxy, n-propoxy , isopropoxy, n-butoxy, an isobutoxy, sec-butoxy and tert-butoxy.

Examples of "aryl" of the "arylalkyl " related to the modified form of B may include aryl having 6 to 12 carbon atoms such as phenyl, 1-naphthyl, 2-naphthyl and biphenyl. The "aryl" of the "arylalkyl" related to the modified form of B may be substituted, and the aryl of the arylalkyl is substituted by 1 to 3 same or different substituents which can be selected from the group consisting of halogen, alkyl, alkoxy, cyano and nitro. Examples of the "halogen" which is a substituent for the "aryl" may include fluorine, chlorine, bromine and iodine. Examples of the "alkyl " which is a substituent for the "aryl " may include straight-chain or branched-chain alkyl having 1 to 4 carbon atoms such as methyl, ethyl, n-propyl, isopropyl, n-butyl, isobutyl, sec-butyl, and tert-butyl. Examples of the "alkoxy" which is a substituent for the "aryl" may include straight-chain or branched-chain alkoxy having 1 to 4 carbon atoms such as methoxy, ethoxy, n-propoxy, isopropoxy, n-butoxy, isobutoxy, sec-butoxy and tert-butoxy.

Examples of the "acyl" related to the modified form of B may include straight-chain or branched-chain alkanoyl having 1 to 6 carbon atoms and aroyl having 7 to 13 carbon atoms. Examples thereof may include formyl, acetyl, propionyl, butyryl, isobutyryl, valeryl, hexanoyl, benzoyl and naphthoyl. The "acyl" related to the modified form of B may be substituted, and the acyl is substituted by 1 to 3 same or different substituents which can be selected from the group consisting of halogen, an alkyl , an alkoxyl , cyano and nitro. Examples of the "halogen" which is a substituent for the "acyl" may include fluorine, chlorine, bromine and iodine. Examples of the "alkyl" which is a substituent for the "acyl" may include straight-chain or branched-chain alkyl having 1 to 4 carbon atoms such as methyl, ethyl, n-propyl, isopropyl, n-butyl, isobutyl, sec-butyl, and a tert-butyl. Examples of the "alkoxy" which is a substituent for the "acyl" may include straight-chain or branched-chain alkoxy having 1 to 4 carbon atoms such as methoxy, ethoxy, n-propoxy, isopropoxy, n-butoxy, isobutoxy, sec-butoxy and tert-butoxy. For example, when B is adenine, cytidine or guanine, by substituting amino with acyl, the acyl plays a role as a protecting group. Specific examples may include 4-anisoyl for adenine and cytidine, and isobutyryl for guanine.

The compound of the present invention can be used as a free acid form as such, however, it can be used in a salt form by a conventional method. Examples of the "salt" may include alkali metal salts such as a sodium salt and a potassium salt, alkaline earth metal salts such as a calcium salt, organic tertiary amine salts such as triethylamine and pyridine.

Specific examples of the compound of the present invention may include the following (1) to (8) ribonucleic acid compounds:
(1) 2-cyanoethyl-2-chlorophenyl 5'-O-levulinyl-2'-O-(2-tetrahydrofuranyl)-N⁴-(4-anisoyl)cytidin3'-yl phosphate;
(2) 2-chlorophenyl 5'-O-levulinyl-2'-O-(2-tetrahydrofuranyl)-N⁴-(4-anisoyl)cytidin-3'-yl phosphate;
(3) 2-cyanoethyl-2-chlorophenyl 5'-O-levulinyl-2'-O-(2-tetrahydrofuranyl)uridin-3'-yl phosphate;
(4) 2-chlorophenyl 5'-O-levulinyl-2'-O-(2-tetrahydrofuranyl)uridin-3'-yl phosphate;
(5) 2-cyanoethyl-2-chlorophenyl 5'-O-levulinyl-2'-O-(2-tetrahydrofuranyl)-N⁶-(4-anisoyl)adenosin-3'-yl phosphate;
(6) 2-chlorophenyl 5'-O-levulinyl-2'-O-(2-tetrahydrofuranyl)-N⁶-(4-anisoyl)adenosin-3'-yl phosphate;
(7) 2-cyanoethyl-2-chlorophenyl 5'-O-levulinyl-2'-O-(2-tetrahydrofuranyl)-N²-isobutyrylguanosin-3'-yl phosphate; and
(8) 2-chlorophenyl 5'-O-levulinyl-2'-O-(2-tetrahydrofuranyl)-N²-isobutyrylguanosin-3'-yl phosphate.

Further, the present invention may include a method for producing a ribonucleic acid compound, in which the hydroxyl at the 5'-position of ribose is regioselectively levulinylated, having any of the following steps (A) to (C).
(A) A method for producing a ribonucleic acid compound represented by the following general formula (3), comprising regioselectively levulinylating the hydroxyl at the 5'-position by allowing a levulinylating agent and a lipase to act on a ribonucleic acid compound represented by the following general formula (2). 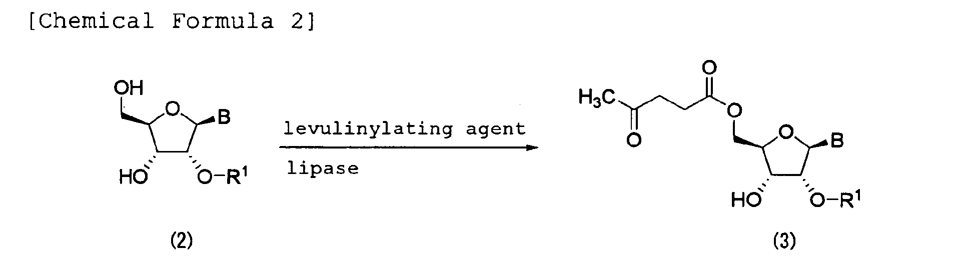 In the formula, B and R¹ are the same as defined above.

(B) A method for producing a ribonucleic acid compound represented by the following general formula (1a) by allowing a phosphorylating reagent to act on a ribonucleic acid compound represented by the following general formula (3) produced by a production method including the step of regioselectively levulinylating the hydroxyl at the 5'-position by allowing a levulinylating agent and a lipase to act on a ribonucleic acid compound represented by the following general formula (2). 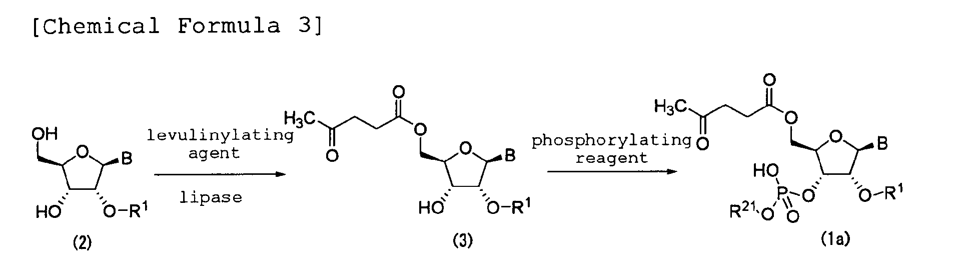 In the formula, B, R¹ and R²¹ are the same as defined above.

(C) A method for producing a ribonucleic acid compound represented by the following general formula (1b) by allowing a phosphorylating reagent and a reagent for protecting a phosphate group to act on a ribonucleic acid compound represented by the following general formula (3) produced by a production method including the step of regioselectively levulinylating the hydroxyl at the 5'-position by allowing a levulinylating agent and a lipase to act on a ribonucleic acid compound represented by the following general formula (2). 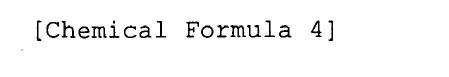 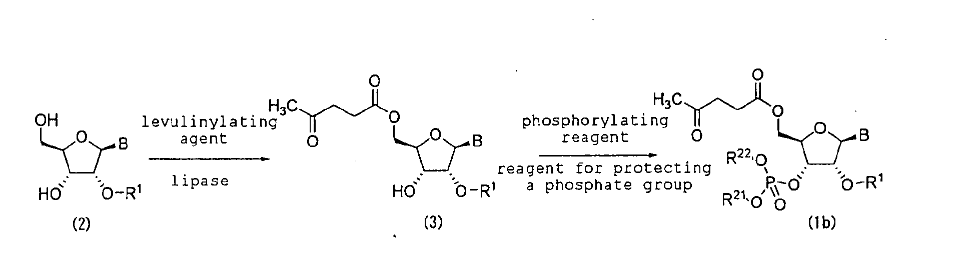 In the formula, B, R¹ and R²¹ are the same as defined above. R²² represents alkyl which may be substituted.
Examples of the "alkyl" and the substituent for the alkyl related to R²² may include the same ones as illustrated in the explanation of the R²⁰.
Examples of the "lipase" according to the present invention may include lipases suitable for the respective substrates. Preferred examples may include Candida antarctica lipase B (Novozym 435: manufactured by Novo Nordisk) and Alcaligenes triacylglycerol lipase (LIPASE-QL: manufactured by Meito Sangyo. Co., LTD.).
Examples of the "levulinylating agent" according to the present invention may include levulinic acid, levulinic anhydride, levulinic acid ester and levulinoyl halide. Examples of the "levulinic acid ester" may include methyl levulinate, ethyl levulinate and vinyl levulinate. Examples of the "levulinoyl halide" may include levulinoyl fluoride, levulinoyl chloride, levulinoyl bromide, and levulinoyl iodide. In particular, levulinic anhydride is preferred.
Examples of the "phosphorylating reagent" may include 2-chlorophenyl phosphoroditriazolide, 2-chlorophenyl-O,O-bis(l-benzotriazolyl)phosphate and 2-chloro-4-tert-butylphenyl phosphoroditriazolide.
Examples of the "reagent for protecting a phosphate group" may include 3-hydroxypropionitril and 2,2,2-trichloroethanol.

Further, the present invention may include a liquid-phase synthesis method for an oligonucleotide compound represented by the following general formula (4) having the following steps (a) to (f). 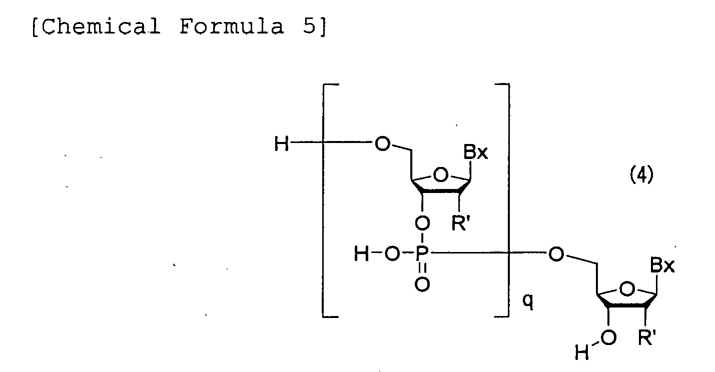 (In the formula, each Bx independently represents adenine, guanine, cytosine, uracil or thymine or a modified form thereof.
q represents an integer in the range from 1 to 100. q may be an integer in the range from 10 to 50, and more preferably, it may be an integer in the range from 15 to 30.
At least one of R' is hydroxyl and the others represent independently H or hydroxyl.)
The modified form of Bx is substituted by 1 to 3 same or different substituents which can be selected from the group consisting of halogen, alkyl, arylalkyl, alkoxy, hydroxyl, amino, a monoalkylamino group, a dialkylamino group, carboxyl, cyano and nitro. Examples of the "halogen", "alkyl", "alkoxy", "arylalkyl" and "aryl" related to the modified form of Bx may include the same ones as illustrated in the explanation of B.

(a) The step of producing a ribonucleic acid compound represented by the following general formula (3) by regioselectively levulinylating the hydroxyl at the 5'-position by allowing a levulinylating agent and a lipase to act on a ribonucleic acid compound represented by the following general formula (2). 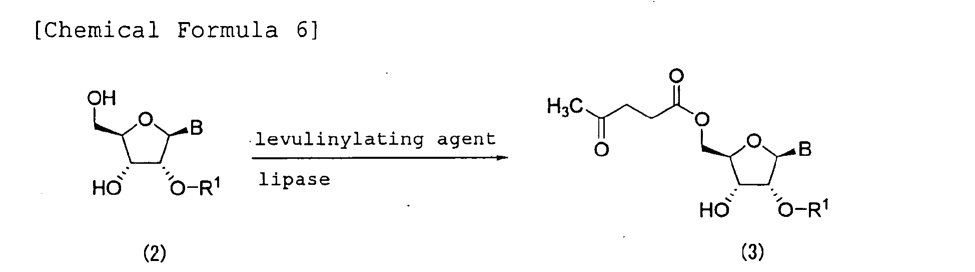 (In the formula, B and R¹ are the same as defined above. The "lipase" and the "levulinylating agent" are also the same as defined above.)

(b) The step of producing a ribonucleic acid compound represented by the following general formula (1a) by phosphorylating the hydroxyl at the 3'-position by allowing a phosphorylating reagent to act on a ribonucleic acid compound (3) produced by the step (a). 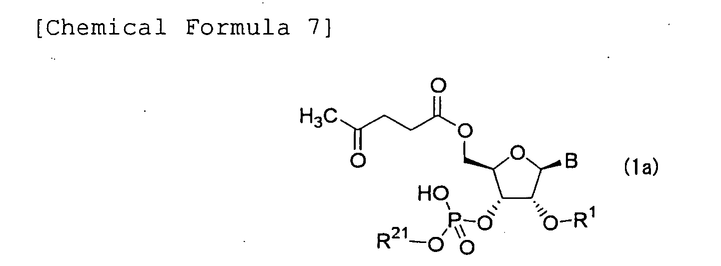 (In the formula, B, R¹ and R²¹ are the same as defined above. The "phosphorylating reagent" is also the same as defined above.)

(c) Separately from the step (b), the step of producing a ribonucleic acid compound represented by the following general formula (1b) by allowing a phosphorylating reagent and a reagent for protecting a phosphate group to act on a ribonucleic acid compound (3) produced by the step (a). 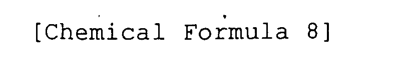 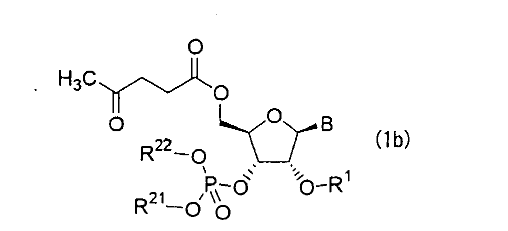 (In the formula, B, R¹, R²¹, R²², the "phosphorylating reagent" and the "reagent for protecting a phosphate group" are the same as defined above.)

(d) The step of producing a ribonucleic acid compound represented by the following general formula (5) by removing levulinyl that protects the hydroxyl at the 5'-position of ribose of the ribonucleic acid compound (1b) produced by the step (c): 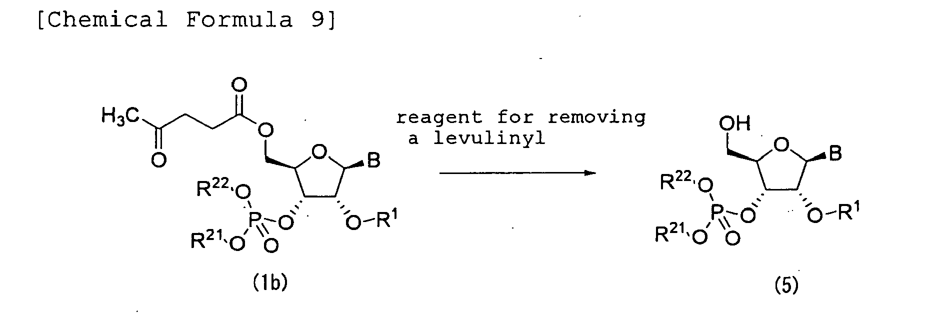 (In the formula, B, R¹, R²¹ and R²² are the same as defined above. Examples of the "reagent for removing levulinyl" according to the present invention may include a solution of 0.5 M hydrazine monohydrate/pyridine-acetic acid (4:1) .)

(e) The step of stepwise oligomerization using as a monomer component, at least one of the ribonucleic acid compounds (1a) and (5) produced by the steps (b) and (d), respectively.
The "monomer component" refers to a deoxyribonucleic acid compound, a ribonucleic acid compound or a modified form thereof constituting an oligonucleotide compound.
Examples of the "modified form" related to the "monomer component" may include the same ones as illustrated above.
The "stepwise oligomerization" may include a condensation step and a selective deprotection step for producing an oligonucleotide compound having a nucleotide polymerization degree of 2 to 100 or an oligonucleotide block having a nucleotide polymerization degree of 2 to 100. Examples of the condensation step may include a condensation step of nucleotide monomer blocks, a condensation step of a nucleotide monomer block with an oligonucleotide block having a nucleotide polymerization degree of 2 to 100 and a condensation step of different oligonucleotide blocks having a nucleotide polymerization degree of 2 to 100. Specifically, by condensing nucleotide monomer blocks or oligonucleotide blocks arbitrarily selected depending on the nucleotide polymerization degree of a desired oligonucleotide compound, the oligonucleotide compound can be produced. For example, in the case where an oligonucleotide having a nucleotide polymerization degree of 30 is produced, a production method using a nucleotide monomer block and an oligonucleotide block having a nucleotide polymerization degree of 29, or a production method using an oligonucleotide block having a nucleotide polymerization degree of 5 and an oligonucleotide block having a nucleotide polymerization degree of 25 is conceivable.
The "nucleotide monomer block" is a deoxyribonucleic acid compound, a ribonucleic acid compound or a modified form thereof for producing an oligonucleotide compound. Examples of the "modified form" may include the same ones as illustrated above.
The "oligonucleotide block" is a compound having a nucleotide polymerization degree of 2 to 100 comprising as a monomer component a nucleotide compound, a ribonucleic acid compound or a modified form thereof for synthesizing an oligonucleotide compound. Examples of the "modified form" may include the same ones as illustrated above.
The "nucleotide polymerization degree" indicates the total number of nucleotide monomers constituting an oligonucleotide compound.

Examples of the "nucleotide monomer block" and the "oligonucleotide block" to be used in the present invention may include compounds represented by the following general formulae (8) and (9).
The compound represented by the following general formula (8) indicates a nucleotide monomer block (m = 0) or an oligonucleotide block (m = 1 to 99). 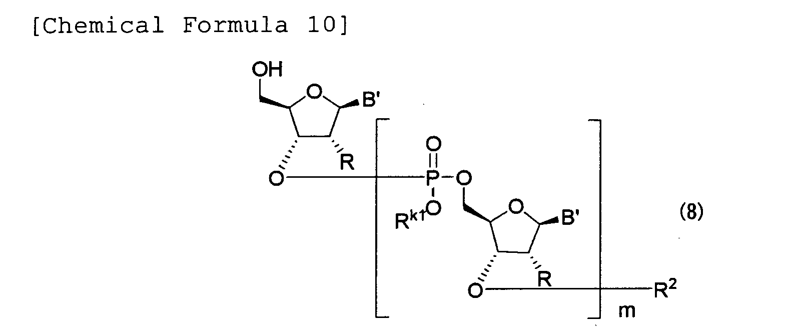 In the formula, each B' independently represents adenine, guanine, cytosine, uracil or thymine or a modified form thereof.
Examples of the substituent related to the "modified form" of each B' may include the same ones as illustrated in the explanation of B.
R² represents acyl or a phosphate group which may be substituted.
Each R^{k1} represents aryl which may be substituted or a monocyclic or a bicyclic heterocyclic group which may be substituted. Examples of the "aryl", the substituent related to the "aryl", the " bicyclic heterocyclic group", the substituent related to the "bicyclic heterocyclic group" of R^{k1} may include the same ones as illustrated in the explanation of R²¹.
m represents an integer in the range from 0 to 99.
Examples of the "acyl" related to R² may include straight-chain or branched-chain alkanoyl having 1 to 6 carbon atoms and aroyl having 7 to 13 carbon atoms. Examples thereof may include formyl, acetyl, propionyl, a butyryl, isobutyryl, valeryl, hexanoyl, benzoyl and naphthoyl. The "acyl" related to R² may be substituted, and the acyl is substituted by 1 to 3 same or different substituents which can be selected from the group consisting of halogen, alkyl, alkoxy, cyano and nitro. Examples of the "halogen" which is a substituent for the "acyl" may include fluorine, chlorine, bromine and iodine. Examples of the "alkyl " which is a substituent for the "acyl" may include straight-chain or branched-chain alkyl having 1 to 4 carbon atoms such as methyl, ethyl , n-propyl, isopropyl, n-butyl, isobutyl, sec-butyl, and tert-butyl. Examples of the "alkoxy" which is a substituent for the "acyl" may include straight-chain or branched-chain alkoxy having 1 to 4 carbon atoms such as methoxy, ethoxy, n-propoxy, isopropoxy, n-butoxy, isobutoxy, sec-butoxy and tert-butoxy.
Examples of the substituent for the "phosphate group which may be substituted" related to R² may include an aryl which may be substituted, a monocyclic or bicyclic heterocyclic group which may be substituted and an alkyl which may be substituted. Examples of the "aryl", the substituent for the aryl, the "monocyclic or bicyclic heterocyclic group which may be substituted" and the substituent for the heterocyclic group may include the same ones as illustrated in the explanation of R²¹. Examples of the "alkyl" and the substituent for the alkyl may include the same ones as illustrated in the explanation of R²².
Each R represents H or hydroxyl substituted with a protecting group which can be removed at 90% or more at a temperature in the range from 0°C to 60°C under acidic conditions at a pH value from 2 to 4 within 24 hours. Preferably, it is H or hydroxyl substituted with a protecting group which can be removed at 90% or more at a temperature in the range from 15°C to 40°C under acidic conditions at a pH value from 2 to 4 within 24 hours. More preferably, it is H or hydroxyl substituted with 2-tetrahydrofuranyl or 1,3-dioxolan-2-yl.

The compound represented by the following general formula (9) indicates a nucleotide monomer block (n = 1) or an oligonucleotide block (n = 2 to 100). 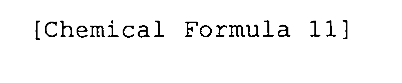 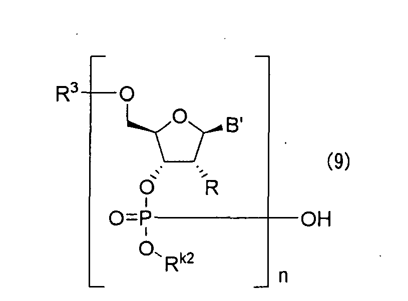 In the formula, each B' and each R are the same as defined above.
Each R^{k2} independently represents aryl which may be substituted or a monocyclic or bicyclic heterocyclic group which may be substituted. Examples of the "aryl", the substituent for the aryl , the "monocyclic or bicyclic heterocyclic group which may be substituted" and the substituent for the heterocyclic group related to R^{k2} may include the same ones as illustrated in the explanation of R²¹.
R³ represents levulinyl or 4,4'-dimethoxytrityl.
n represents an integer in the range from 1 to 100.

The "condensation step" can be classified into the following 4 types.
(i) A condensation step of a nucleotide monomer block with a nucleotide monomer block.
   It is a condensation step of nucleotide monomer blocks in which at least one of the nucleotide monomer blocks is a ribonucleic acid compound (1a) or (5).
(ii) A condensation step (1) of a nucleotide monomer block with an oligonucleotide block having a nucleotide polymerization degree of 2 to 100.
   It is a condensation step of a nucleotide monomer block with an oligonucleotide block having a nucleotide polymerization degree of 2 to 100, in which the nucleotide monomer block is a ribonucleic acid compound (1a) or (5) and the oligonucleotide block having a nucleotide polymerization degree of 2 to 100 is a compound (8) or (9).
(iii) A condensation step (2) of a nucleotide monomer block with an oligonucleotide block having a nucleotide polymerization degree of 2 to 100.
   It is a condensation step of a nucleotide monomer block with an oligonucleotide block having a nucleotide polymerization degree of 2 to 100, in which the nucleotide monomer block is a compound (8) or (9) and the oligonucleotide block (8) or (9) having a nucleotide polymerization degree of 2 to 100 contains as a monomer component, at least one of the ribonucleic acid compounds (1a) and (5).
(iv) A condensation step of different oligonucleotide blocks having a nucleotide polymerization degree of 2 to 99.
   It is a condensation step of different oligonucleotide blocks (8) and (9) having a nucleotide polymerization degree of 2 to 99, in which the oligonucleotide block (8) or (9) having a nucleotide polymerization degree of 2 to 99 contains as a monomer component, at least one of the ribonucleic acid compounds (1a) and (5).
The above-mentioned condensation steps shown in (i) to (iv) are a step of condensing the hydroxyl at the 5'-position of a nucleotide monomer block or an oligonucleotide block with the phosphate group substituted for the hydroxyl at the 3'-position of the other nucleotide monomer block or oligonucleotide block by allowing a condensing reagent to act on.
Examples of the "condensing reagent" according to the present invention may include 1-(2-mesitylenesulfonyl)-3-nitro-1,2,4-triazole, 2,4,6-trimethylbenzenesulfonyl tetrazole and 1-(2,4,6-triisopropylbenzenesulfonyl)-3-nitro-1,2,4-triazole.

The "selective deprotection step" refers to a step of removing, for example, levulinyl which is a protecting group substituted for the hydroxyl at the 5'-position of a monomer component at the 5' end or, for example, cyanoethyl which is a protecting group on a phosphate group substituted for the hydroxyl at the 3'-position of a monomer component at the 3' end in the oligonucleotide compound produced by the condensation step.
The "reagent for removing levulinyl" is the same as defined above.
Examples of the "reagent for removing cyanoethyl " which is a protecting group on a phosphate group substituted for the hydroxyl at the 3'-position of ribose according to the present invention may include a solution of pyridine/triethylamine/water (3:1:1).

Specific examples of the protected oligonucleotide compound to be produced may include a compound represented by the following general formula (6). 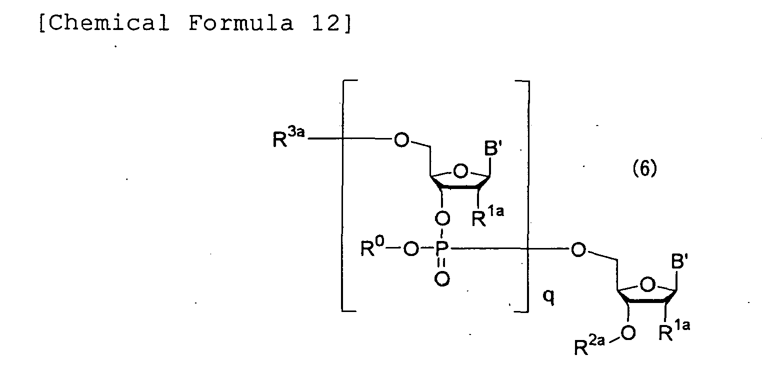 In the formula, each B' is independently the same as defined above.
Each R⁰ independently represents H, an aryl which may be substituted or a monocyclic or bicyclic heterocyclic group which may be substituted. Preferably, it is independently H, phenyl which may be substituted or a monocyclic or bicyclic heterocyclic group which may be substituted. More preferably, it is 2-chlorophenyl or 2-chloro-4-tert-butylphenyl. Examples of the "aryl" and the "monocyclic or bicyclic heterocyclic group which may be substituted" and the substituents for the aryl and the heterocyclic group related to R⁰ may include the same ones as illustrated in the explanation of R²¹.
R^{3a} represents H, levulinyl or 4,4'-dimethoxytrityl.
q is the same as defined above.
At least one of R^{1a} is hydroxyl substituted with a protecting group which can be removed at 90% or more at a temperature in the range from 0°C to 60°C under acidic conditions at a pH value from 2 to 4 within 24 hours, and the others independently represent H or hydroxyl substituted with a protecting group which can be removed at 90% or more at a temperature in the range from 0°C to 60°C under acidic_conditions at a pH value from 2 to 4 within 24 hours. Preferably, it is H or hydroxyl substituted with a protecting group which can be removed at 90% or more at a temperature in the range from 15°C to 40°C under acidic conditions at a pH value from 2 to 4 within 24 hours. More preferably, H or hydroxyl substituted with 2-tetrahydrofuranyl and 1,3-dioxolan-2-yl can be exemplified.
R^{2a} represents acyl or a phosphate group represented by the following general formula (7). 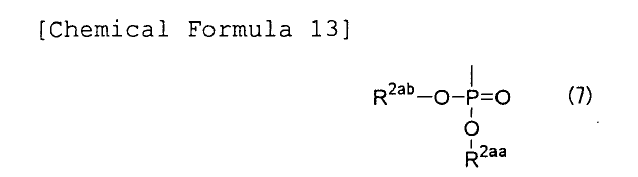 In the formula, R^{2aa} represents aryl which may be substituted or a monocyclic or bicyclic heterocyclic group which may be substituted. R^{2ab} represents H or alkyl which may be substituted.
Examples of the "aryl", the substituent for the aryl, the "monocyclic or bicyclic heterocyclic which may be substituted" and the substituent for the heterocyclic group related to R^{2aa} may include the same ones as illustrated in the explanation of R²¹. Preferably, 2-chlorophenyl and 2-chlorophenyl-4-tert-butylphenyl can be exemplified.
Examples of the "alkyl" and the substituent for the alkyl related to R^{2ab} may include the same ones as illustrated in the explanation of R²².
Examples of the "acyl" related to R^{2a} may include the same ones as illustrated in the explanation of R².

(f) The step of deprotecting all the protecting groups of the protected oligonucleotide compound (6) produced by the step (e).
As the reagent for removing each protecting group, the following reagents are exemplified.
1) N¹, N¹, N³, N³-tetramethylguanidine and pyridine-2-carboxaldoxime
2) concentrated aqueous ammonia solution
3) acetic acid buffer (pH = 3 to 4)
4) 80% aqueous acetic acid solution
5) dilute hydrochloric acid solution(pH = 2 to 4)
Note that as for the reagents 3) to 5), it is only necessary to use one of them, however, it is preferred to use the reagent 3).
Examples of the "acetic acid buffer" may include a sodium acetate/acetic acid buffer and a tetramethylethylenediamine/acetic acid buffer.

### Best Mode for Carrying Out the Invention

The present invention can be carried out as follows.
In the following production method, in the case where a raw material has a substituent that is not desired to be reacted such as hydroxyl, amino and carboxyl, it is general that the raw material is protected with a protecting group such as benzoyl and 4-anisoyl by a known method in advance and then is used in reaction. After the reaction, the protecting group can be removed by a known method such as catalytic reduction, alkali treatment, acid treatment or the like.

Step (a) : (Production method in which the hydroxyl at the 5'-position of ribose of a ribonucleic acid is levulinylated) 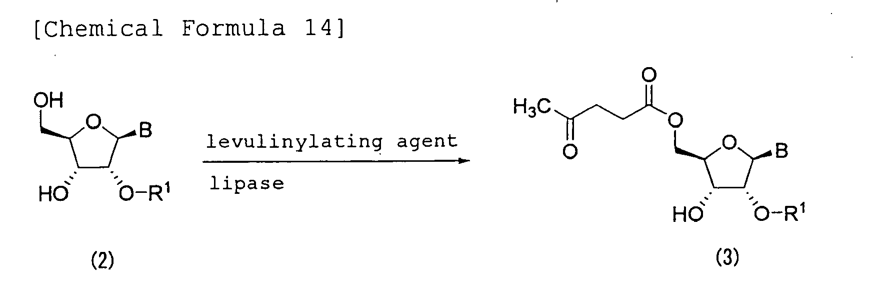 In the formula, B, R¹, the "lipase" and the "levulinylating agent" are the same as defined above.
In the method of introducing levulinyl into the hydroxyl at the 5'-position of a ribonucleic acid in the present invention, a product (3) in which the desired hydroxyl at the 5'-position of ribose is levulinylated can be selectively obtained by suspending a compound (2) and an excess levulinylating agent in an appropriate solvent and reacting the mixture with a lipase.
The amount of the levulinylating agent to be used is preferably in the range from 1- to 20-fold molar amount, more preferably from 1- to 10-fold molar amount based on the compound (2).
The solvent to be used is not particularly limited as long as it is not involved in the reaction, however, examples thereof may include ethers such as tetrahydrofuran, diethyl ether and 1,4-dioxane, amides such as N,N-dimethylformamide and N,N-dimethylacetamide, nitriles such as acetonitrile and propionitrile, hydrocarbons such as benzene and toluene and mixed solvents thereof.
The reaction temperature in the reaction is preferably in the range from 20°C to 50°C which is an optimal temperature for the enzymatic activity. The reaction time varies depending on the type of raw material to be used and the reaction temperature, however, in general, it is suitably in the range from 30 minutes to 100 hours.

Step (b): (Production method in which the hydroxyl at the 3'-position of ribose of a ribonucleic acid is phosphorylated, Part 1) 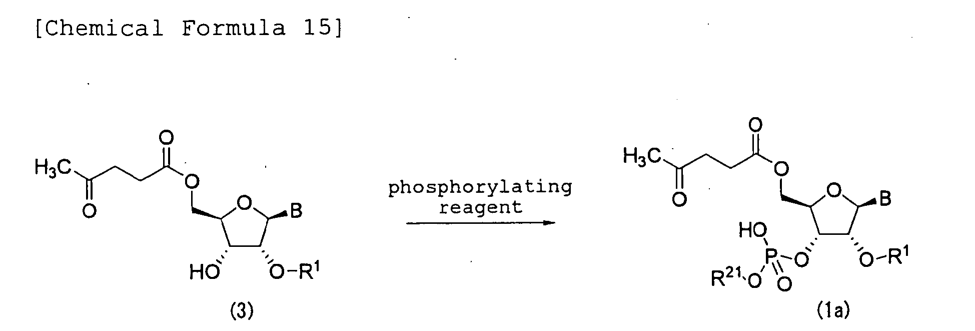 In the formula, B, R¹, R²¹ and the "phosphorylating reagent" are the same as defined above.
In the present invention, a highly protected ribonucleic acid compound (1a) can be produced by allowing a phosphorylating reagent to act on the ribonucleic acid compound (3) in the presence of an organic base. The solvent to be used is not particularly limited as long as it is not involved in the reaction, however, examples thereof may include ethers such as tetrahydrofuran, diethyl ether and 1,4-dioxane, nitriles such as acetonitrile and propionitrile, hydrocarbons such as benzene and toluene and mixed solvents thereof. The phosphorylating reagent is in an amount ranging from 1- to 20-fold molar amount, more preferably from 1- to 10-fold molar amount based on the compound (3).
The reaction temperature is suitably in the range, for example, from -20°C to 100°C, preferably from 0°C to 80°C, more preferably from 5°C to 30°C. The reaction time varies depending on the type of raw material to be used and the reaction temperature, however, in general, it is suitably in the range from 30 minutes to 100 hours.

Step (c): (Production method in which the hydroxyl at the 3'-position of ribose of a ribonucleic acid is phosphorylated, Part 2) 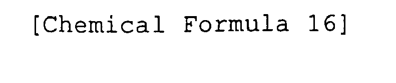 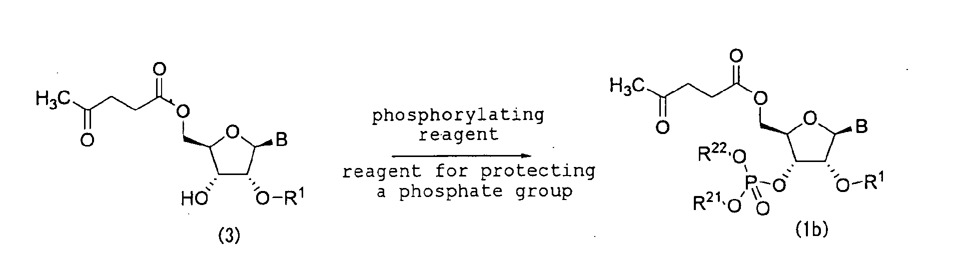 In the formula, B, R¹, R²¹, R²² the "phosphorylating reagent" and the "reagent for protecting a phosphate group" are the same as defined above.
In the present invention, a highly protected ribonucleic acid compound (1b) can be produced by allowing a phosphorylating reagent and a reagent for protecting a phosphate group to act on the ribonucleic acid compound (3) in the presence of an organic base. The solvent to be used is not particularly limited as long as it is not involved in the reaction, however, examples thereof may include ethers such as tetrahydrofuran, diethyl ether and 1,4-dioxane, nitriles such as acetonitrile and propionitrile, hydrocarbons such as benzene and toluene and mixed solvents thereof. The amount of the phosphorylating reagent to be used is in the range from 1-to 20-fold molar amount, more preferably from 1- to 10-fold molar amount based on the compound (3). The amount of the reagent for protecting a phosphate group to be used is in the range from 1- to 20-fold molar amount, more preferably from 1- to 10-fold molar amount based on the compound (3). The reaction temperature is suitably in the range, for example, from -20°C to 100°C, preferably from 0°C to 80°C, more preferably from 5°C to 30°C. The reaction time varies depending on the type of raw material to be used and the reaction temperature, however, in general, it is suitably in the range from 30 minutes to 100 hours.

Step (d): (Step of removing levulinyl that protects the hydroxyl at the 5'-position of ribose) 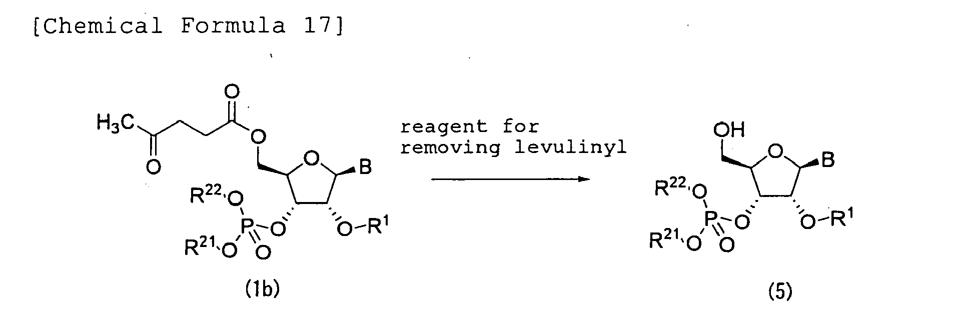 In the formula, B, R¹, R²¹, R²² and the "reagent for removing levulinyl" are the same as defined above.
A compound (5) can be produced by adding a reagent for removing levulinyl to the compound (1b). This reaction is carried out in the absence of a solvent or in a suitable solvent. The solvent to be used is not particularly limited as long as it is not involved in the reaction, however, examples thereof may include alcohols such as methanol and ethanol, ethers such as tetrahydrofuran, diethyl ether and 1,4-dioxane, amides such as N,N-dimethylformamide and N,N-dimethylacetamide, nitriles such as acetonitrile and propionitrile, hydrocarbons such as benzene and toluene and mixed solvents thereof. The amount of the reagent for removing levulinyl to be used is in the range from 1- to 20-fold molar amount, more preferably from 1- to 10-fold molar amount based on the compound (1b). The reaction temperature is suitably in the range, for example, from - 20°C to 100°C, preferably from 0°C to 80°C, more preferably from 15°C to 65°C. The reaction time varies depending on the type of raw material to be used and the reaction temperature, however, in general, it is suitably in the range from 30 minutes to 100 hours.

Hereinafter, a liquid-synthesis method for an oligonucleotide compound will be described.
Step (e) : (Method for stepwise production of oligonucleotide, Part 1)
This step is stepwise oligomerization using as a monomer component, at least one of the ribonucleic acid compounds represented by the general formulae (1a) and (5) in the stepwise production process of an oligonucleotide compound. The stepwise oligomerization is composed of a condensation step and a selective deprotection step.
In the condensation step, by allowing a condensing reagent to act on, an oligonucleotide compound or an oligonucleotide block can be produced. In general, the reaction is carried out in an appropriate solvent in the presence of an excess organic amine. The solvent to be used is not particularly limited as long as it is not involved in the reaction, however, examples thereof may include ethers such as tetrahydrofuran, diethyl ether and 1,4-dioxane, nitriles such as acetonitrile and propionitrile, hydrocarbons such as benzene and toluene, organic amines such as pyridine and mixed solvents thereof. Examples of the organic amine may include pyridine and the like. The amount of the condensing reagent to be used is in the range from 1- to 20-fold molar amount, more preferably from 1- to 10-fold molar amount based on the ribonucleic acid compound represented by the formula (1a) or (5), or a nucleotide monomer block or an oligonucleotide block represented by the formula (8) or (9). The reaction temperature in the reaction is suitably in the range, for example, from -20°C to 100°C, preferably from 0°C to 80°C, more preferably from 5°C to 30°C. The reaction time varies depending on the type of raw material to be used and the reaction temperature, however, in general, it is suitably in the range from 30 minutes to 100 hours.
The selective deprotection step is a step of producing an oligonucleotide block selectively deprotected by allowing the reagent for deprotection to act on the oligonucleotide block produced in the above-mentioned condensation step in such a manner that the resulting oligonucleotide block can be used in the next condensation step. Specifically, it is a step of removing, for example, levulinyl which is a protecting group substituted for the hydroxyl at the 5'-position of a nucleic acid molecule at the 5' end or, for example, cyanoethyl which is a protecting group on a phosphate group substituted for the hydroxyl at the 3'-position of a nucleic acid at the 3' end in the oligonucleotide block produced in the above-mentioned condensation step.
The "reagent for removing levulinyl" and the "reagent for removing cyanoethyl" are the same as defined above.
In particular, in a step of producing an oligonucleotide compound finally, it is preferred to use a nucleotide monomer block or an oligonucleotide block represented by the general formula (8) or (9) in which R³ is provided with a protecting group with extremely high lipophilicity such as a 4,4'-dimethoxytrityl in the condensation step. It is advantageous in the step of separating impurities derived from nucleic acid compounds in a purification step after the reaction, and deprotection can be carried out under the same conditions as those for the protecting group at the 2'-position of each ribose in the deprotection step, therefore, there is an advantage to enable the reduction of a step.

Step (f): (Method for stepwise production of oligonucleotide, Part 2) 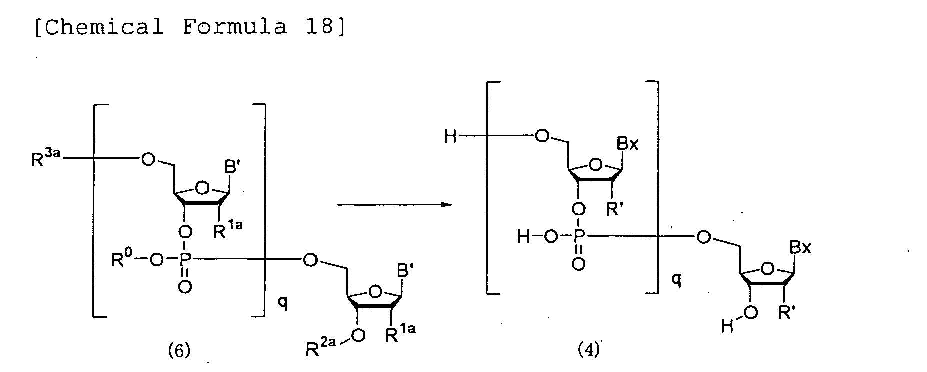 In the formula, each Bx, B' , R', R⁰, R^{1a}, R^{2a}, R^{3a} and q are the same as defined above.
A compound (4) can be obtained by adding a reagent for deprotecting each protecting group to a compound (6) and allowing a reaction to proceed. The solvent to be used is not particularly limited as long as it is not involved in the reaction, however, examples thereof may include ethers such as tetrahydrofuran, diethyl ether and 1,4-dioxane, organic amines such as pyridine, 28% aqueous ammonia solution and mixed solvents thereof. Examples of the organic amine may include pyridine and the like. The reaction temperature in the reaction is suitably in the range, for example, from -20°C to 100°C, preferably from 0°C to 80°C, more preferably from 10°C to 60°C. The reaction time varies depending on the type of raw material to be used and the reaction temperature, however, in general, it is suitably in the range from 30 minutes to 100 hours.
The compounds according to the present invention can be isolated and purified from the above-mentioned reaction mixture by using a standard separation and purification technique such as extraction, concentration, neutralization, filtration, centrifugation, recrystallization, silica gel column chromatography, thin-layer chromatography, reverse-phase ODS column chromatography, hydrophobic column chromatography, ion-exchange column chromatography, gel filtration column chromatography, dialysis, ultrafiltration or the like.

### [Examples]

Hereinafter, the present invention will be described in more detail with reference to Reference examples and Examples, however, the present invention is not limited thereto.

### Reference example 1

### 2'-O-(2-Tetrahydrofuranyl)-N⁴-(4-anisoyl)cytidine

### Step 1: Synthesis of 3',5'-O-(1,1,3,3-tetraisopropyl-disiloxan-1,3-diyl)-2'-O-trimethylsilyl-N⁴-(4-anisoyl)cytidine

Cytidine (20.0 g) was suspended in 200 ml of anhydrous pyridine, 27.2 g of 1,3-dichloro-1,1,3,3-tetraisopropyl disiloxane was added dropwise thereto, and the mixture was stirred for 10 minutes. Then, the temperature was returned to room temperature and the mixture was stirred for 90 minutes. To the reaction solution, 20.6 g of trimethylsilylchloride was added dropwise at 0°C, and the mixture was stirred at room temperature for 2 hours. Then, 28.1 g of 4-anisoylchloride was added dropwise thereto, and the mixture was stirred at room temperature for 1.5 hours. Then, 60 ml of ice water was added to the reaction solution and the mixture was stirred for 10 minutes, and then, 25 ml of aqueous ammonia solution was added thereto, and the mixture was stirred at room temperature for 30 minutes. Then, the reaction solution was poured in 400 ml of water and extraction was performed with dichloromethane. The dichloromethane layer was washed with a saturated aqueous sodium bicarbonate solution and a saturated brine, and then dried and concentrated. The obtained residue was purified by silica gel column chromatography (n-hexane : ethyl acetate = 3 : 1 to 1 : 1), whereby 39.5 g of the above-mentioned compound was obtained.

### Step 2: Synthesis of 3',5'-O-(1,1,3,3-tetraisopropyl-disiloxan-1,3-diyl)-N⁴-(4-anisoyl)cytidine

3',5'-O-(1,1,3,3-Tetraisopropyl-disiloxan-1,3-diyl)-2'-O-trimethylsilyl-N⁴-(4-anisoyl)cytidine (39.5 g) was dissolved in 200 ml of dichloromethane and 200 ml of methanol, and 7.2 g of pyridinium p-toluene sulfonate was added thereto and the mixture was stirred at 65°C. Then, triethylamine was added and the mixture was neutralized and then concentrated. The residue was purified by silica gel column chromatography (n-hexane : ethyl acetate = 1 : 2 to 1 : 4), whereby 34.0 g of the above-mentioned compound was obtained.

### Step 3: Synthesis of 3',5'-O-(1,1,3,3-tetraisopropyl-disiloxan-1,3-diyl)-2'-O-(2-tetrahydrofuranyl)-N⁴-(4-anisoyl)cytidine

3',5'-O-(1,1,3,3-Tetraisopropyl-disiloxan-1,3-diyl)-N⁴-(4-anisoyl)cytidine (30.0 g) and 10.2 g of 2, 3-dihydrofurane were dissolved in 150 ml of anhydrous tetrahydrofuran, and 5.62 g of camphorsulfonic acid was added in small portions thereto at 0°C, and then, the mixture was stirred at room temperature for 1 hour. The mixture was poured in a saturated aqueous sodium bicarbonate solution and extraction was performed with ethyl acetate. Then, washing was performed with a saturated brine, and then, drying and concentration were performed. The residue was purified by silica gel column chromatography (n-hexane : ethyl acetate = 1 : 3), whereby 31.5 g of the above-mentioned compound was obtained.

### Step 4: Synthesis of 2'-O-(2-tetrahydrofuranyl)-N⁴-(4-anisoyl)cytidine

3',5'-O-(1,1,3,3-Tetraisopropyl-disiloxan-1,3-diyl)-2'-O-(2-tetrahydrofuranyl)-N⁴-(4-anisoyl)cytidine (33.2 g) was dissolved in 80 ml of anhydrous tetrahydrofuran, and 97 ml of a solution of 1 M tetrabutylammonium fluoride in tetrahydrofuran was added dropwise thereto over 15 minutes, and then, the mixture was stirred at room temperature for 1 hour. The reaction solution was concentrated, and a saturated aqueous sodium bicarbonate solution was added thereto. Then, extraction was performed with dichloromethane, and washing was performed with a saturated brine, and then, drying and concentration were performed. The residue was purified by silica gel column chromatography (3% methanol/dichloromethane to 5% methanol/dichloromethane), whereby 7.11 g of the objective compound was obtained.

### Reference example 2

### Thymidine 3'-(2-chlorophenoxy)phosphoryl thymodine 3'-acetate

### Step 1: Synthesis of 3'-O-acetylthymidine

5'-O-(4,4'-dimethoxytrityl)thymidine (2.0 g) was subjected to azeotropic distillation with pyridine, and 0.75 g of acetic anhydride was added thereto at 0°C, and then, the mixture was stirred at room temperature for 21 hours. After the concentration, the resulting mixture was dissolved in chloroform and washed with a saturated aqueous sodium bicarbonate solution and a saturated brine, and then dried and concentrated. The residue was washed with n-hexane and then dried, whereby 1.84 g of 5'-O-(4,4'-dimethoxytrityl)-3'-O-acetylthymidine was obtained.
The resulting 5'-O-(4,4'-dimethoxytrityl)-3'-O-acetylthymidine (1.84 g) was dissolved in 30 ml of dichloromethane, and 16.5 ml of 0.38 M benzenesulfonic acid in methanol was added dropwise thereto at 0°C, and then, the mixture was stirred for 15 minutes. To the reaction solution, a saturated aqueous sodium bicarbonate solution was added and extraction was performed with dichloromethane, and then, drying and concentration were performed. The residue was washed with ether and then dried, whereby 0.32 g of the above-mentioned compound was obtained.

### Step 2: Synthesis of 2-chlorophenyl 5'-O-(4,4'-dimethoxytrityl)thymidin-3'-yl phosphate triethylamine salt

5'-O-(4,4'-Dimethoxytrityl)thymidine (2.0 g) was subjected to azeotropic distillation with pyridine, and 20 ml of a solution of 0.28 M 2-chlorophenyl phosphoroditriazolide in dioxane was added thereto, and the mixture was stirred at room temperature for 1 hour. Then, 8 ml of 50% aqueous pyridine solution was added thereto and the mixture was concentrated. To the residue, 20 ml of a 0.2 M aqueous triethylammonium bicarbonate solution was added, and dichloromethane extraction was performed. The dichloromethane layer was washed twice with 20 ml of a 0.2 M aqueous triethylammonium bicarbonate solution, and then dried and concentrated. The residue was crystallized from ethylacetate/n-hexane, and the resulting crystal was dried, whereby 1.88 g of the above-mentioned compound was obtained.

### Step 3: Synthesis of 5'-O-(4,4'-dimethoxytrityl)thymidine 3'-(2-chlorophenoxy)phosphoryl thymidine 3'-acetate

2-Chlorophenyl 5'-O-(4,4'-dimethoxytrityl)thymidin-3'-yl phosphate triethylamine salt and 3'-O-acetylthymidine were subjected to azeotropic distillation with pyridine, and the resulting substance was dissolved in 3 ml of anhydrous pyridine. Then, 0.71 g of 1-(2-mesitylenesulfonyl)-3-nitro-1,2,4-triazole (MSNT) was added thereto, and the mixture was stirred at room temperature for 1 hour. To the reaction solution, a saturated aqueous sodium bicarbonate solution was added, and extraction was performed with dichloromethane, and then drying and concentration were performed. The residue was purified by silica gel column chromatography (2% methanol/chloroform to 4% methanol/chloroform containing 0.1% pyridine), whereby 1.06 g of the above-mentioned compound was obtained.

### Step 4: Synthesis of thymidine 3'-(2-chlorophenoxy)phosphoryl thymidine 3'-acetate

5'-O-(4,4'-Dimethoxytrityl)thymidine 3'-(2-chlorophenoxy)phosphoryl thymidine 3'-acetate (1.05 g) was dissolved in 10 ml of dichloromethane, and 5.2 ml of a solution of 0.38 M benzenesulfonic acid in methanol was added dropwise thereto at 0°C, and then, the mixture was stirred for 30 minutes. The reaction solution was washed with a saturated aqueous sodium bicarbonate solution and a saturated brine, and then dried and concentrated, whereby 0.69 g of the objective compound was obtained.

### Reference example 3

### 2-Chlorophenyl 5'-O-(4,4'-Dimethoxytrityl)-2'-O-(2-etrahydrofuranyl)-N²-isobutyrylguanosin-3'-yl phosphate triethylamine salt

### Step 1: Synthesis of N²-isobutyrylguanosine

Guanosine (20.0 g) was suspended in 540 ml of chloroform and 70 ml of pyridine, and 48 ml of isobutyric chloride was added dropwise thereto, and then the mixture was stirred at room temperature for 2 hours. While the reaction solution was stirred and cooled with ice water, 1 N hydrochloric acid was added and extraction was performed with dichloromethane. The organic layer was washed with water and then with a 1 N aqueous sodium carbonate solution, and then dried with anhydrous magnesium sulfate and then concentrated under reduced pressure. The residue was subjected to azeotropic distillation with toluene, and the resulting substance was dissolved in 54 ml of methanol. Then, 54 ml of a solution of 28% sodium methoxide in methanol was added dropwise slowly while stirring and cooling with ice water, and the mixture was stirred for 10 minutes. The reaction solution was neutralized with Dowex (H form), filtered to remove Dowex, and washed well with methanol. The mother liquid and washing solution were combined and concentrated under reduced pressure. The residue was dissolved in a small amount of methanol, and added dropwise slowly to acetone to obtain powder. The obtained powder was collected, whereby 17.9 g of the above-mentioned compound was obtained.
MS m/z: 354 [MH]⁺

### Step 2: Synthesis of 3',5'-0-(1,1,3,3-tetraisopropyldisiloxan-1,3-diyl)-N²-isobutyrylguanosine

N²-Isobutyrylguanosine (16.9 g) was dissolved in 100 ml of pyridine, and 16.6 g of 1,3-dichloro-1,1,3,3-tetraisopropyl disiloxane was added dropwise thereto while stirring and cooling with ice water, and then the mixture was stirred at room temperature for 4 hours. The reaction solution was added dropwise to water, and the obtained crystal was collected and then dried. The crude crystal was recrystallized from ethanol, whereby 30.3 g of the above-mentioned compound was obtained.
Melting point: 179-183°C
MS m/z: 596 [MH]⁺

### Step 3: Synthesis of 2'-O-,(2-tetrahydrofuranyl)-N²⁻isobutyrylguanosine

3',5'-O-(1,1,3,3-Tetraisopropyl disiloxan-1,3-diyl)-N²-isobutyrylguanosine (30.0 g) was dissolved in 330 ml of tetrahydrofuran, and 7.83 g of 2,3-dihydrofurane and 6.49 g of (+)-camphorsulfonic acid were added thereto, and then the mixture was stirred at room temperature for 2 hours. Then, while stirring and cooling with ice water, 55.0 ml of a solution of 1 M tetra-n-butylammonium fluoride in tetrahydrofuran was added dropwise thereto, and the mixture was stirred for 10 minutes. The reaction solution was concentrated under reduced pressure and purified by silica gel column chromatography (1% methanol/dichloromethane to 2% methanol/dichloromethane), whereby 20.2 g of the above-mentioned compound was obtained.
Melting point: 178-180°C
MS m/z: 424 [MH]⁺

### Step 4: Synthesis of 5'-O-(4,4'-dimethoxytrityl)-2'-O-(2-tetrahydrofuranyl)-N²-isobutyrylguanosine

2'-O-(2-Tetrahydrofuranyl)-N²-isobutyrylguanosine (0.70 g) was subjected to azeotropic distillation with pyridine and suspended in 15 ml of tetrahydrofuran and 1.34 ml of pyridine. Then, 0.61 g of 4,4'-dimethoxytrityl chloride was added thereto while stirring and cooling with ice water, and the mixture was stirred at room temperature for 4 hours. The reaction solution was added to a saturated aqueous sodium bicarbonate solution, and dichloromethane extraction was performed. The organic layer was washed with a saturated brine, and then dried and concentrated. The residue was purified by silica gel column chromatography (dichloromethane to 1% methanol/dichloromethane), whereby 1.00 g of the above-mentioned compound was obtained.

### Step 5: Synthesis of 2-chlorophenyl 5'-O-(4,4'-dimethoxytrityl)-2'-O-(2-tetrahydrofuranyl)-N²-isobutyrylguanosin-3'-yl phosphate triethylamine salt

The same reaction was carried out using the compound synthesized in the step 2 of Reference example 2 instead of 5'-O-(4,4'-dimethoxytrityl)thymidine in the step 2 of Reference example 2, whereby the objective compound was obtained.

### Example 1

### 5'-O-levulinyl-2'-O-(2-tetrahydrofuranyl)-N⁴-(4-anisoyl)cytidine

2'-O-(2-Tetrahydrofuranyl)-N⁴-(4-anisoyl)cytidine (7.0 g) was suspended in 280 ml of anhydrous tetrahydrofuran, and 10.1 g of levulinic anhydride and 2.78 g of Novozym 435 were added thereto, and then the mixture was shaked at room temperature for 25 hours. Then, 3.0 g of levulinic anhydride and 1.0 g of Novozym 435 were further added thereto, and then the mixture was shaked at room temperature for 72 hours. The reaction solution was filtered through celite, and washed with dichloromethane. Then, to the filtrate, a saturated aqueous sodium bicarbonate solution was added, and the mixture was stirred for 15 minutes. Then, separation, drying and concentration were performed. The residue was purified by silica gel column chromatography (2% methanol/dichloromethane), whereby 6.02 g of the objective compound was obtained.

### Example 2

### 2-Cyanoethyl 2-chlorophenyl 5'-O-levulinyl-2'-O-(2-tetrahydrofuranyl)-N⁴-(4-anisoyl)cytidin-3'-yl phosphate

To 3.0 g of 5'-O-levulinyl-2'-O-(2-tetrahydrofuranyl)-N⁴-(4-anisoyl)cytidine, 19.5 ml of a solution of 0.56 M 2-chlorophenyl phosphoroditriazolide in dioxane was added and the substance was dissolved. Then, 0.90 g of 1-methylimidazole was added thereto and the mixture was stirred at room temperature for 1 hour. Then, 1.56 g of 3-hydroxypropionitrile was added thereto, and the mixture was stirred at room temperature for 2 hours. Then, 5. ml of 50% aqueous pyridine solution was added thereto at 0°C, and the mixture was stirred for 5 minutes. Then, the reaction solution was added to a saturated aqueous sodium bicarbonate solution and extraction was performed with dichloromethane. Then, washing was performed with a saturated aqueous sodium bicarbonate solution and a saturated brine, and drying and concentration were performed. The residue was purified by silica gel column chromatography (n-hexane : ethyl acetate = 20 : 80 to 15 : 85), whereby 3.16 g of the objective compound was obtained. MS m/z: 789.3 [MH]⁺

### Example 3

### 2-Chlorophenyl 5'-O-levulinyl-2'-O-(2-tetrahydrofuranyl)-N⁴-(4-anisoyl)cytidin-3'-yl phosphate triethylamine salt

To 3.0 g of 5'-O-levulinyl-2'-O-(2-tetrahydrofuranyl)-N⁴-(4-anisoyl)cytidine, 19.5 ml of a solution of 0.56 M 2-chlorophenyl phosphoroditriazolide in dioxane was added and the substance was dissolved. Then, 0.90 g of 1-methylimidazole was added thereto and the mixture was stirred at room temperature for 1 hour. Then, 10 ml of 50% aqueous pyridine solution was added thereto at 0°C, and the mixture was stirred for 10 minutes and concentrated. To the residue, 35 ml of a 0.2 M aqueous triethylammonium bicarbonate solution was added, and dichloromethane extraction was performed. The dichloromethane layer was washed twice with 15 ml of a 0.2 M aqueous triethylammonium bicarbonate solution, and then dried and concentrated. The residue was purified by silica gel column chromatography (10% methanol/dichloromethane to 15% methanol/dichloromethane), whereby 4.03 g of the objective compound was obtained.

### Example 4

### 5'-O-Levulinyl-2'-O-(2-tetrahydrofuranyl)-N²-isobutyrylguanosine

2'-O-(2-Tetrahydrofuranyl)-N²-isobutyrylguanosine (5.70 g) was suspended in 270 ml of acetonitrile, and 8.66 g of levulinic anhydride and 2.40 g of Novozym AS were added thereto, and then the mixture was stirred at room temperature for 15 hours. The reaction solution was filtered and Novozym AS was removed, which was washed well with dichloromethane. The mother liquid and washing solution were combined, and a substantially the same volume of a saturated aqueous sodium bicarbonate solution was added thereto and the mixture was vigorously stirred overnight. The organic layer was washed with a saturated aqueous sodium bicarbonate solution and then with a saturated brine, and then dried and concentrated. The residue was dissolved in a small amount of dichloromethane, and added dropwise slowly to a mixed solvent containing equal amount of n-hexane and diethyl ether, to obtain powder. The obtained powder was collected, washed with a mixed solvent containing equal amount of n-hexane and diethyl ether, whereby 6.80 g of the above-mentioned compound was obtained. MS m/z: 522 [MH]⁺

### Example 5

### 2-Cyanoethyl 2-chlorophenyl 5'-O-levulinyl-2'-O-(2-tetrahydrofuranyl)-N²-isobutyrylguanosin-3'-yl phosphate

The same reaction was carried out using 5'-O-levulinyl-2'-O-(2-tetrahydrofuranyl)-N²⁻isobutyrylguanosine instead of 5'-O-levulinyl-2'-O-(2-tetrahydrofuranyl)-N⁴-(4-anisoyl)cytidine in Example 2, whereby the objective compound was obtained.

### Example 6

### 2-Chlorophenyl 5'-O-levulinyl-2'-O-(2-tetrahydrofuranyl)-N²-isobutyrylguanosin-3'-yl phosphate triethylamine salt

The same reaction was carried out using 5'-O-levulinyl-2'-O-(2-tetrahydrofuranyl)-N²⁻isobutyrylguanosine instead of 5'-O-levulinyl-2'-O-(2-tetrahydrofuranyl)-N⁴-(4-anisoyl)cytidine in Example 3, whereby the objective compound was obtained.

### Example 7

### 2-Cyanoethyl 2-chlorophenyl 5'-O-levulinyl-2'-O-(2-tetrahydrofuranyl)uridin-3'-yl phosphate

The same reaction was carried out using 5'-O-levulinyl-2'-O-(2-tetrahydrofuranyl)uridine instead of 5'-O-levulinyl-2'-O-(2-tetrahydrofuranyl)-N⁴-(4-anisoyl)cytidine in Example 2, whereby the objective compound was obtained.

### Example 8

### 2-Chlorophenyl 5'-O-levulinyl-2'-O-(2-tetrahydrofuranyl)uridin-3'-yl phosphate triethylamine salt

The same reaction was carried out using 5'-O-levulinyl-2'-O-(2-tetrahydrofuranyl)uridine instead of 5'-O-levulinyl-2'-O-(2-tetrahydrofuranyl)-N⁴-(4-anisoyl)cytidine in Example 3, whereby the objective compound was obtained.

### Example 9

### 2-Cyanoethyl 2-chlorophenyl 5'-O-levulinyl-2'-O-(2-tetrahydrofuranyl)-N⁶-(4-anisoyl)adenosin-3'-yl phosphate

The same reaction was carried out using 5'-O-levulinyl-2'-O-(2-tetrahydrofuranyl)-N⁶-(4-anisoyl) adenosine instead of 5'-O-levulinyl-2'-O-(2-tetrahydrofuranyl)-N⁴-(4-anisoyl)cytidine in Example 2, whereby the objective compound was obtained.

### Example 10

### 2-Chlorophenyl 5'-O-levulinyl-2'-O-(2-tetrahydrofuranyl)-N⁶-(4-anisoyl)adenosin-3'-yl phosphate triethylamine salt

The same reaction was carried out using 5'-O-levulinyl-2'-O-(2-tetrahydrofuranyl)-N⁶-(4-anisoyl)adenosine instead of 5'-O-levulinyl-2'-O-(2-tetrahydrofuranyl)-N⁴-(4-anisoyl)cytidine in Example 3, whereby the objective compound was obtained.

### Example 11

### Adenylyl-[3'→5']-uridylyl-[3'→5']-guanylyl-[3'→5']-cytidylyl-[3'→5']-thymidylyl-[3'→5']-thymidine

### (A) Oligonucleotide block A: 2'-O-(2-Tetrahydrofuranyl)-N²-isobutyrylguanosine 3'-(2-chlorophenoxy)phosphoryl 2'-O-(2-tetrahydrofuranyl)-N⁴-(4-anisoyl)cytidine 3'- (2-chlorophenoxy)phosphoryl thymdine 3'-(2-chlorophenoxy)phosphoryl thymidine 3'-acetate

### Step 1: Synthesis of 2-cyanoethyl 2-chlorophenyl 2'-O-(2-tetrahydrofuranyl)-N⁴-(4-anisoyl)cytidin-3'-yl phosphate

To 3.16 g of 2-cyanoethyl 2-chlorophenyl 5'-O-levulinyl-2'-O-(2-tetrahydrofuranyl)-N⁴-(4-anisoyl)cytidin-3'-yl phosphate, 40 ml of a solution of 0.5 M hydrazine/pyridine-acetic acid (4:1) was added and the substance was dissolved, and then the mixture was stirred at room temperature for 20 minutes. Then, 10 ml of acetone was added thereto at 0°C and the mixture was stirred for 5 minutes. Then, the mixture was diluted with 200 ml of ethyl acetate and poured in a saturated aqueous sodium bicarbonate solution to separate the solution. The ethyl acetate layer was washed with a saturated aqueous sodium bicarbonate solution, and then dried and concentrated. The residue was purified by silica gel column chromatography (dichloromethane to 5% methanol/dichloromethane), whereby 2.43 g of the above-mentioned compound was obtained.

### Step 2: Synthesis of 5'-O-levulinyl-2'-O-(2-tetrahydrofuranyl)-N²-isobutyrylguanosine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

2-Chlorophenyl 5'-O-levulinyl-2'-O-(2-tetrahydrofuranyl)-N2-isobutyrylguanosin-3'-yl phosphate triethylamine salt (1.95 g) and 1.38 g of 2-cyanoethyl 2-chlorophenyl 2'-O-(2-tetrahydrofuranyl)-N'-(4-anisoyl)cytidin-3'-yl phosphate were subjected to azeotropic distillation with pyridine, and then dissolved in 3 ml of anhydrous pyridine. Then, 1.42 g of 1-(2-mesitylenesulfonyl)-3-nitro-1,2,4-triazole (MSNT) was added thereto and the mixture was stirred at room temperature for 1 hour. To the reaction solution, a saturated aqueous sodium bicarbonate solution was added, and extraction was performed with dichloromethane, and then drying and concentration were performed. The residue was purified by silica gel column chromatography (2% methanol/chloroform to 4% methanol/chloroform containing 0.1% pyridine), whereby 2.21 g of the above-mentioned compound was obtained.

### Step 3: Synthesis of 5'-O-levulinyl-2'-O-(2-tetrahydrofuranyl)-N²-isobutyrylguanosine 3'-(2-chlorophenoxy)phosphoryl 2'-O-(2-tetrahydrofuranyl)-N⁴-(4-anisoyl)cytidine 3'-[O-(2-chlorophenyl)phosphate] triethylamine salt

To 2.2 g of the compound synthesized in the step 2 of (A) of Example 11, 32 ml of a solution of pyridine/triethylamine/water (3:1:1) was added, and the mixture was stirred at room temperature for 30 minutes. The reaction solution was concentrated and then dried, whereby 2.43 g of the above-mentioned compound was obtained.

### Step 4: Synthesis of 5'-O-levulinyl-2'-O-(2-tetrahydrofuranyl)-N²-isobutyrylguanosine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

The same reaction as the step 2 of (A) of Example 11 was carried out using the compound of the step 3 of (A) of Example 11 and the compound of Reference example 3, whereby the objective compound was obtained.

### Step 5: Synthesis of 2'-O-(2-tetrahydrofuranyl)-N²-isobutyrylguanosine 3'-(2-chlorophenoxy)phosphoryl 2'-O-(2-tetrahydrofuranyl)-N⁴-(4-anisoyl)cytidine 3'-(2-chlorophenoxy)phosphoryl thymdine 3'-(2-chlorophenoxy)phosphoryl thymidine 3'-acetate

The same reaction was carried out using 5'-O-levulinyl-2'-O-(2-tetrahydrofuranyl)-N²⁻isobutyrylguanosine 3'-(2-chlorophenoxy)phosphoryl 2'-O-(2-tetrahydrofuranyl)-N⁴-(4-anisoyl) cytidine 3'-(2-chlorophenoxy)phosphoryl thymdine 3'-(2-chlorophenoxy)phosphoryl thymidine 3'-acetate instead of 2-cyanoethyl 2-chlorophenyl 5'-O-levulinyl-2'-O-(2-tetrahydrofuranyl)-N⁴-(4-anisoyl)cytidin-3'-yl phosphate in the step 1 of (A) of Example 11, whereby the objective compound was obtained.

### (B) Oligonucleotide block B: 5'-O-(4,4-Dimethoxytrityl)-2'-O-(2-tetrahydrofuranyl)-N⁶-(4-anisoyl)adenosine 3'-(2-chlorophenoxy)phosphoryl 2'-O-(2-tetrahydrofuranyl)uridine 3'-[O-(2-cyanoethyl)(2-chlorophenyl) phosphate]

### Step 1: Synthesis of 2-cyanoethyl 2-chlorophenyl 2'-O-(2-tetrahydrofuranyl)uridin-3'-yl phosphate

The same reaction was carried out using 2-cyanoethyl 2-chlorophenyl 5'-O-levulinyl-2'-O-(2-tetrahydrofuranyl)uridin-3'-yl phosphate instead of 2-cyanoethyl 2-chlorophenyl 5'-O-levulinyl-2'-O-(2-tetrahydrofuranyl)-N⁴-(4-anisoyl)cytidin-3'-yl phosphate in the step 1 of (A) of Example 11, whereby the objective compound was obtained.

### Step 2: Synthesis of 5'-O-levulinyl-2'-O-(2-tetrahydrofuranyl)-N⁶-(4-anisoyl)adenosine 3'-(2-chlorophenoxy)phosphoryl 2'-O-(2-tetrahydrofuranyl)uridine 3'-[O-(2-cyanoethyl)(2-chlorophenyl)phosphate]

The same reaction as the step 2 of (A) of Example 11 was carried out using the compound synthesized in Example 10 and the compound synthesized in the step 1 of (B) of Example 11, whereby the objective compound was obtained.

### Step 3: Synthesis of 2'-O-(2-tetrahydrofuranyl)-N⁶-(4-anisoyl)adenosine 3'-(2-chlorophenoxy)phosphoryl 2'-O-(2-tetrahydrofuranyl)uridine 3'-[O-(2-cyanoethyl)(2-Chlorophenyl)phosphate]

The same reaction was carried out using 5'-O-levulinyl-2'-O-(2-tetrahydrofuranyl)-N⁶-(4-anisoyl)adenosine 3'-(2-chlorophenoxy)phosphoryl 2'-O-(2-tetrahydrofuranyl)uridine 3'-[O-(2-cyanoethyl)(2-chlorophenyl)phosphate] instead of 2-cyanoethyl 2-chlorophenyl 5'-O-levulinyl-2'-O-(2-tetrahydrofuranyl)-N⁴⁻(4-anisoyl)cytidin-3'-yl phosphate in the step 1 of (A) of Example 11, whereby the objective compound was obtained.

### Step 4: Synthesis of 5'-O-(4,4-dimethoxytrityl)-2'-O-(2-tetrahydrofuranyl)-N⁶-(4-anisoyl)adenosine 3'-(2-chlorophenoxy)phosphoryl 2'-O-(2-tetrahydrofuranyl)uridin 3'-[O-(2-cyanoethyl)(2-chlorophenyl)phosphate]

The compound synthesized in the step 3 of (B) of Example 11 (0.33 g) was subjected to azeotropic distillation with pyridine, and then dissolved in 1.3 ml of tetrahydrofuran. Then, 0.22 ml of pyridine and 0.19 g of 4,4'-dimethoxytrityl chloride were added thereto and the mixture was stirred at room temperature. After 2 hours, 0.19 g of 4,4'-dimethoxytrityl chloride was further added thereto and the mixture was stirred for 1 hour. The reaction solution was added to a saturated aqueous sodium bicarbonate solution, and extraction was performed with ethyl acetate. The organic layer was washed with a saturated brine, and then dried and concentrated. The residue was purified by silica gel column chromatography (dichloromethane to 2% methanol/dichloromethane), whereby 0.42 g of the above-mentioned compound was obtained.

### C) Adenylyl-[3'→5']-uridylyl-[3'→5']-guanylyl-[3'→5']-cytidylyl-[3'→5']-thymidylyl-[3'→5']-thymidine

### Step 1: Synthesis of completely protected compound

To 0.035 g of the oligonucleotide block B, a mixed solvent of pyridine, triethyamine and water (3:1:1, 0.22 ml) was added, and the mixture was stirred at room temperature for 20 minutes. The reaction solution was concentrated under reduced pressure, and the resulting substance was subjected to azeotropic distillation with pyridine. Then, 0.030 g of the oligonucleotide block A was added thereto, and the mixture was subjected to azeotropic distillation with pyridine. The residue was dissolved in 0.06 ml of pyridine, and 0.055 g of 1-(2-mesitylenesulfonyl)-3-nitro-1,2,4-triazole (MSNT) was added thereto, and then, the mixture was stirred at room temperature for 1 hour. To the reaction solution, 2 ml of 50% aqueous pyridine solution was added while stirring and cooling with ice water, and then a saturated aqueous sodium bicarbonate solution was added. Then, ethyl acetate extraction was performed, and washing was performed with a saturated brine, and then, drying and concentration were performed. The residue was purified by silica gel column chromatography (dichloromethane to 2% methanol/dichloromethane). The obtained colorless foam-like substance was dissolved in a small amount of dichloromethan, which was added dropwise to diethyl ether to form powder. The obtained powder was collected, and washed with diethyl ether, whereby 0.029 g of 5'-O-(4,4-dimethoxytrityl)-2'-O-(2-tetrahydrofuranyl)-N⁶-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N2-isobutyrylguanosine 3'-(2-chlorophenoxy)phosphoryl 2'-O-(2-tetrahydrofuranyl)-N⁴-(4-anisoyl)cytidine 3'-(2-chlorophenoxy)phosphoryl thymdine 3'-(2-chlorophenoxy)phosphoryl thymidine 3'-acetate was obtained.
Melting point: 179-183°C

### Step 2: Deprotection and purification steps

To 0.005 g of the compound synthesized in the step 1 of (C) of Example 11, 50% aqueous dioxane solution of 0.30 M pyridine-2-carboxaldoxime and N¹,N¹,N³,N³-tetramethyl guanidine was added, and the mixture was stirred at room temperature for 40 hours. The reaction solution was concentrated under reduced pressure, and the resulting substance was dissolved in 1 ml of pyridine. Then, 2 ml of 28% aqueous ammonia solution was added thereto, and the mixture was sealed and left at 55°C for 15 hours. After standing to cool, the mixture was concentrated under reduced pressure, and to the residue, acetone was added. Then, the mixture was centrifuged, whereby light yellow powder was obtained. The obtained powder was purified by reverse-phase ODS column chromatography (acetonitrile / 0.05 M-TEAA buffer).

To the obtained light yellow powder compound, 0.05 ml of 0.1 M acetic acid-tetramethylethylenediamine buffer (pH 3.88) was added, and the mixture was left at 60°C for 30 minutes. The reaction solution was diluted with 0.30 ml of 0.05 M TEAA buffer, and passed through a reverse-phase ODS (2 g of Sep-Pak VaC, water, and then 50% aqueous acetonitrile solution), whereby the objective compound, adenylyl-[3'→5']-uridylyl-[3'→5']-guanylyl-[3'→5']-cytidylyl-[3'→5']-thymidylyl-[3'→5']-thymidine (50.4 OD (A₂₆₀)), was obtained.

### Example 12

### Cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-thimidilyl-[3'→5']-thymidine

### Step 1: Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

2-Chlorophenyl 5'-O-levulinyl-2'-O-(2-tetrahydrofuranyl)-N⁴-(4-anisoyl)cytidin-3'-yl phosphate triethylamine salt (1.32 g) and 0.77 g of 2-cyanoethyl-2-chlorophenyl 2'-O-(2-tetrahydrofuranyl)-N⁴-(4-anisoyl)cytidin-3'-yl phosphate were subjected to azeotropic distillation with pyridine, and then dissolved in 4 ml of anhydrous pyridine. Then, 0.99 g of 1-(2-mesitylenesulfonyl)-3-nitro-1,2,4-triazole (MSNT) was added thereto and the mixture was stirred at room temperature for 1 hour. To the reaction solution, a saturated aqueous sodium bicarbonate solution was added, and extraction was performed with ethyl acetate, and then drying and concentration were performed. The residue was purified by silica gel column chromatography (1% methanol/dichloromethane to 5% methanol/dichloromethane containing 0.1% pyridine), whereby 1.39 g of the above-mentioned compound was obtained.

### Step 2: Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

To 90 mg of the compound synthesized in the step 1 of Example 12, 0.5 ml of a solution of pyridine/triethylamine/water (3:1:1) was added, and the mixture was stirred at room temperature for 30 minutes. The reaction solution was concentrated and then, 30 mg of thymidine 3'-(2-chlorophenoxy)phosphoryl thymidine 3'-acetate was added thereto, and the mixture was subjected to azeotropic distillation with pyridine. The resulting substance was dissolved in 0.5 ml of anhydrous pyridine, and 38 mg of 1-(2-mesitylenesulfonyl)-3-nitro-1,2,4-triazole (MSNT) was added thereto, and then, the mixture was stirred at room temperature for 1 hour. To the reaction solution, a saturated aqueous sodium bicarbonate solution was added, and extraction was performed with ethyl acetate, and then drying and concentration were performed. The residue was purified by silica gel column chromatography (2% methanol/dichloromethane to 5% methanol/dichloromethane containing 0.1% pyridine), whereby 77 mg of the above-mentioned compound was obtained.

### Step 3: Synthesis of 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

To 550 mg of the compound synthesized in the step 1 of Example 12, 6 ml of a solution of 0.5 M hydrazine/pyridine-acetic acid (4:1) was added and the compound was dissolved, and then the mixture was stirred at room temperature for 30 minutes. Then, 1 ml of acetone was added thereto at 0°C and the mixture was stirred for 5 minutes. Then, the mixture was diluted with ethyl acetate and poured in a saturated aqueous sodium bicarbonate solution to separate the solution. The ethyl acetate layer was washed with a saturated aqueous sodium bicarbonate solution, and then dried and concentrated. The residue was purified by silica gel column chromatography (2% methanol/ dichloromethane to 5% methanol/dichloromethane), whereby 465 mg of the above-mentioned compound was obtained.

### Step 4 : Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

To 726 mg of the compound synthesized in the step 1 of Example 12, 6 ml of a solution of pyridine/triethylamine/water (3:1:1) was added, and the mixture was stirred at room temperature for 15 minutes. The reaction solution was concentrated and then, 450 mg of the compound synthesized in the step 3 of Example 12 was added thereto, and the mixture was subjected to azeotropic distillation with pyridine. The resulting substance was dissolved in 2 ml of anhydrous pyridine, and 302 mg of 1-(2-mesitylenesulfonyl)-3-nitro-1,2,4-triazole (MSNT) was added thereto, and then, the mixture was stirred at room temperature for 2 hours. To the reaction solution, a saturated aqueous sodium bicarbonate solution was added, and extraction was performed with ethyl acetate, and then drying and concentration were performed. The residue was purified by silica gel column chromatography (2% methanol/dichloromethane to 5% methanol/dichloromethane containing 0.1% pyridine), whereby 860 mg of the above-mentioned compound was obtained.

### Step 5: Synthesis of 2'-O-(2-tetrahydrofuranyl)-N⁴-(4-anisoyl) cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

To 70 mg of the compound synthesized in the step 2 of Example 12, 0.34 ml of a solution of 0.5 M hydrazine/pyridine-acetic acid (4:1) was added and the compound was dissolved, and then the mixture was stirred at room temperature for 20 minutes. Then, 1 ml of acetone was added thereto at 0°C and the mixture was stirred for 5 minutes. Then, the mixture was diluted with ethyl acetate and poured in a saturated aqueous sodium bicarbonate solution to separate the solution. The ethyl acetate layer was washed with a saturated aqueous sodium bicarbonate solution, and then dried and concentrated. The residue was purified by silica gel column chromatography (2% methanol/dichloromethane to 5% methanol/dichloromethane), whereby 57 mg of the above-mentioned compound was obtained.

### Step 6: Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine thymidine 3'-(2-chlorophenoxy)phosphoryl thymidine 3'-acetate

To 113 mg of the compound synthesized in the step 4 of Example 12, 2 ml of a solution of pyridine/triethylamine/water (3:1:1) was added, and the mixture was stirred at room temperature for 15 minutes. The reaction solution was concentrated and then, 55 mg of the compound synthesized in the step 5 of Example 12 was added thereto, and the mixture was subjected to azeotropic distillation with pyridine. The resulting substance was dissolved in 0.5 ml of anhydrous pyridine, and 25 mg of 1-(2-mesitylenesulfonyl)-3-nitro-1,2,4-triazole (MSNT) was added thereto, and then, the mixture was stirred at room temperature for 2 hours. To the reaction solution, a saturated aqueous sodium bicarbonate solution was added, and extraction was performed with ethyl acetate, and then drying and concentration were performed. The residue was purified by silica gel column chromatography (2% methanol/dichloromethane to 8% methanol/dichloromethane containing 0.1% pyridine), whereby 90 mg of the above-mentioned compound was obtained.

### Step 7: Synthesis of 2'-O-(2-tetrahydrofuranyl)-N⁴-(4-anisoyl)cytidine 3'-(2-chlorophenoxy)phosphoryl 2'-O-(2-tetrahydrofuranyl)-N'-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

To 240 mg of the compound synthesized in the step 4 of Example 12, 1 ml of a solution of 0.5 M hydrazine/pyridine-acetic acid (4:1) was added and the compound was dissolved, and then the mixture was stirred at room temperature for 30 minutes. Then, 0.5 ml of acetone was added thereto at 0°C and the mixture was stirred for 5 minutes. Then, the mixture was diluted with ethyl acetate and poured in a saturated aqueous sodium bicarbonate solution to separate the solution. The ethyl acetate layer was washed with a saturated aqueous sodium bicarbonate solution, and then dried and concentrated. The residue was purified by silica gel column chromatography (2% methanol/dichloromethane to 5% methanol/dichloromethane), whereby 185 mg of the above-mentioned compound was obtained.

### Step 8: Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N4-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

To 288 mg of the compound synthesized in the step 4 of Example 12, 4 ml of a solution of pyridine/triethylamine/water (3:1:1) was added, and the mixture was stirred at room temperature for 15 minutes. The reaction solution was concentrated and then, 180 mg of the compound synthesized in the step 7 of Example 12 was added thereto, and the mixture was subjected to azeotropic distillation with pyridine. The resulting substance was dissolved in 0.5 ml of anhydrous pyridine, and 63 mg of 1-(2-mesitylenesulfonyl)-3-nitro-1,2,4-triazole (MSNT) was added thereto, and then, the mixture was stirred at room temperature for 1.5 hours. To the reaction solution, a saturated aqueous sodium bicarbonate solution was added, and extraction was performed with ethyl acetate, and then drying and concentration were performed. The residue was purified by silica gel column chromatography (2% methanol/dichloromethane to 6% methanol/dichloromethane containing 0.1% pyridine), whereby 339 mg of the above-mentioned compound was obtained.

### Step 9: Synthesis of 2'-O-(2-tetrahydrofuranyl)-N⁴-(4-anisoyl)cytidine 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thimidine 3'-acetate

To 90 mg of the compound synthesized in the step 6 of Example 12, 1 ml of a solution of 0.5 M hydrazine/pyridine-acetic acid (4:1) was added and the compound was dissolved, and then the mixture was stirred at room temperature for 20 minutes. Then, 0.5 ml of acetone was added thereto at 0°C and the mixture was stirred for 5 minutes. Then, the mixture was diluted with ethyl acetate and poured in a saturated aqueous sodium bicarbonate solution to separate the solution. The ethyl acetate layer was washed with a saturated aqueous sodium bicarbonate solution, and then dried and concentrated. The residue was purified by silica gel column chromatography (2% methanol/dichloromethane to 6% methanol/dichloromethane), whereby 60 mg of the above-mentioned compound was obtained.

### Step 10: Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl) cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

To 105 mg of the compound synthesized in the step 8 of Example 12, 2 ml of a solution of pyridine/triethylamine/water (3:1:1) was added, and the mixture was stirred at room temperature for 15 minutes. The reaction solution was concentrated and then, 58 mg of the compound synthesized in the step 9 of Example 12 was added thereto, and the mixture was subjected to azeotropic distillation with pyridine. The resulting substance was dissolved in 0.5 ml of anhydrous pyridine, and 25 mg of 1-(2-mesitylenesulfonyl)-3-nitro-1,2,4-triazole (MSNT) was added thereto, and then, the mixture was stirred at room temperature for 2 hours. To the reaction solution, a saturated aqueous sodium bicarbonate solution was added, and extraction was performed with dichloromethane, and then drying and concentration were performed. The residue was purified by silica gel column chromatography (2% methanol/dichloromethane to 6% methanol/dichloromethane containing 0.1% pyridine), whereby 80 mg of the above-mentioned compound was obtained.

### Step 11: Synthesis of 5'-O-(4,4-dimethoxytrityl)-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate]

2-Chlorophenyl 5'-O-(4,4-dimethoxytrityl)-2'-O-(2-tetrahydrofuranyl)-N⁴-(4-anisoyl)cytidin-3'-yl phosphate triethylamine salt (107 mg) and 175 mg of the compound synthesized in the step 7 of Example 12 were subjected to azeotropic distillation with pyridine, and the resulting substance was dissolved in 1 ml of anhydrous pyridine. Then, 61 mg of 1-(2-mesitylenesulfonyl)-3-nitro-1,2,4-triazole (MSNT) was added thereto, and the mixture was stirred at room temperature for 1.5 hours. To the reaction solution, a saturated aqueous sodium bicarbonate solution was added, and extraction was performed with ethyl acetate, and then drying and concentration were performed. The residue was purified by silica gel column chromatography (2% methanol/dichloromethane to 6% methanol/dichloromethane containing 0.1% pyridine), whereby 225 mg of the above-mentioned compound was obtained.

### Step 12: Synthesis of 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

To 55 mg of the compound synthesized in the step 10 of Example 12, 1 ml of a solution of 0.5 M hydrazine/pyridine-acetic acid (4:1) was added and the compound was dissolved, and then the mixture was stirred at room temperature for 15 minutes. Then, 0.5 ml of acetone was added thereto at 0°C and the mixture was stirred for 5 minutes. Then, the mixture was diluted with dichloromethane and poured in a saturated aqueous sodium bicarbonate solution to separate the solution. The dichloromethane layer was washed with a saturated aqueous sodium bicarbonate solution, and then dried and concentrated. The residue was purified by silica gel column chromatography (2% methanol/dichloromethane to 6% methanol/dichloromethane), whereby 44 mg of the above-mentioned compound was obtained.

### Step 13: Synthesis of 5'-O-(4,4-dimethoxytrityl)-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

To 27 mg of the compound synthesized in the step 11 of Example 12, 2 ml of a solution of pyridine/triethylamine/water (3:1:1) was added, and the mixture was stirred at room temperature for 15 minutes. The reaction solution was concentrated and then, 44 mg of the compound synthesized in the step 12 of Example 12 was added thereto, and the mixture was subjected to azeotropic distillation with pyridine. The resulting substance was dissolved in 0.5 ml of anhydrous pyridine, and 5 mg of 1-(2-mesitylenesulfonyl)-3-nitro-1,2,4-triazole (MSNT) was added thereto, and then, the mixture was stirred at room temperature for 3 hours. To the reaction solution, a saturated aqueous sodium bicarbonate solution was added, and extraction was performed with dichloromethane, and then drying and concentration were performed. The residue was purified by silica gel column chromatography (2% methanol/dichloromethane to 6% methanol/dichloromethane containing 0.1% pyridine), whereby 42 mg of the above-mentioned compound was obtained.

### Step 14: Deprotection and purification steps

To 20 mg of the compound synthesized in the step 13 of Example 12, 50% aqueous dioxane solution of 0.30 M pyridine-2-carboxaldoxime and N¹,N¹,N³,N³-tetramethyl guanidine was added, and the mixture was stirred at room temperature for 72 hours. The reaction solution was concentrated under reduced pressure, and the resulting substance was dissolved in 1 ml of pyridine. Then, 5 ml of 28% aqueous ammonia solution was added thereto, and the mixture was sealed and left at 55°C for 7 hours, and then left overnight at room temperature. The reaction solution was concentrated, and to the residue, acetone was added. Then, the mixture was centrifuged, whereby light yellow powder was obtained. The obtained powder was purified by reverse-phase ODS column chromatography (acetonitrile/0.05 M-TEAA buffer). To the resulting substance, 0.05 ml of 0.1 M acetic acid-tetramethylethylenediamine buffer (pH 3.88) was added, and the mixture was left at 60°C for 1 hour. The reaction solution was desalted by reverse-phase ODS, whereby the objective compound, cytidylyl-[3'→5']-cytidylyl-[3'→ 5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-cytidylyl-[3'→5']-thymidylyl-[3'→5']-thymidine, was obtained.
MALDI TOF MS: Calculated value: 6344.86, Measured value: 6345.69

### Example 13

### 5'-O-(4,4-Dimethoxytrityl)-2'-O-(2-tetrahydrofuranyl)-N⁴⁻(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4*-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

The same reaction was carried out using the compound of the step 3 of Example 12 instead of the compound synthesized in the step 7 of Example 12 in the step 11 of Example 12, whereby a trimer block was synthesized. Then, by using the trimer block synthesized above instead of the compound synthesized in the step 11 of Example 12 in the step 13 of Example 12, the same reaction was carried out using the compound of the step 5 of Example 12 instead of the compound synthesized in the step 12 of Example 12, whereby the objective oligonucleotide compound was synthesized.

### Example 14

### 5'-O-(4,4-Dimethoxytrityl)-2'-O-(2-tetrahydropyranyl)-N⁴⁻(4-anisoyl)cytidine 3'-(2-chlorophenoxy)phosphoryl 2'-O-(2-tetrahydropyranyl)-N⁴-(4-anisoyl)cytidine 3'-(2-chlorophenoxy)phosphoryl 2'-O-(2-tetrahydropyranyl)-N⁴-(4-anisoyl)cytidine 3'-(2-chlorophenoxy)phosphoryl 2'-O-(2-tetrahydropyranyl)-N⁴-(4-anisoyl)cytidine 3'-(2-chlorophenoxy)phosphoryl 2'-O-(2-tetrahydropyranyl)-N⁴-(4-anisoyl)cytidine 3'-(2-chlorophenoxy)phosphoryl thymidine 3'-(2-chlorophenoxy)phosphoryl thymidine 3'-acetate

By using the same method as in Example 13, the objective oligonucleotide compound was synthesized using a nucleotide monomer block in which the protecting for the hydroxyl at the 2'-position is a 2-tetrahydropyranyl.

### Example 15

By using the compound of Example 13 in which the protecting group for the hydroxyl at the 2'-position is 2-tetrahydrofuranyl and the compound of Example 14 in which the protecting group for the hydroxyl at the 2'-position is 2-tetrahydropyranyl as test compounds and subjecting the compounds to a treatment by a method shown below, deprotection reactions were compared and examined.
To 18 mg of either of the test compounds, 2 ml of a solution of 0.3 M N¹,N¹,N³,N³-tetramethyl guanidine and pyridine-2-carboxaldoxime in dioxane/water (1/1) was added, and the mixture was stirred overnight at room temperature. After the reaction solution was concentrated, the resulting substance was dissolved in 2 ml of pyridine and 10 ml of concentrated aqueous ammonia solution, and then the mixture was stirred overnight at 55°C. After the reaction solution was concentrated, acetone was added thereto. Then, the deposited precipitate was collected by centrifugation and washed 3 times with acetone, and then dried. To 1 mg of the resulting substance, 1 ml of 0.1 M sodium acetate/acetic acid buffer (pH 3.82) was added, and reaction was carried out under the respective deprotection conditions. The reaction was followed by reverse-phase HPLC. The yields (HPLC peak area) of the deprotected compounds are shown in Table 1.

**Table 1**

| Deprotection conditions | Yield (in the case of compound of Example 13) | Yield (in the case of compound of Example 14) |
|---|---|---|
| 20°C / 24 h | 97% | 55% |
| 20°C / 48 h | 97% | 79% |
| 60°C / 1 h | 97% | 85% |

From the above results, in the case where the 2-tetrahydrofuranyl was used as the protecting for the hydroxyl at the 2'-position, the deprotected compound was obtained in a substantially quantitative manner under all the conditions. However, in the case where the 2-tetrahydropyranyl was used as the protecting group for the hydroxyl at the 2'-position, production of the deprotected compound was not sufficient. Therefore, it is found that the 2-tetrahydrofuranyl is apparently useful as the protecting group for the hydroxyl at the 2'-position.

### Example 16 Levulinylation with lipase

### 5'-O-Levulinyl-2'-O-(2-tetrahydrofuranyl)uridine

2'-O-(2-tetrahydrofuranyl)uridine (50 mg) was dissolved in 2.5 ml of anhydrous acetonitrile, and 140 mg of levulinic anhydride and 28 mg of either of lipases were added, and then the mixture was stirred at room temperature for 16 hours. By using reverse-phase HPLC, the yield of 5'-O-levulinyl-2'-O-(2-tetrahydrofuranyl)uridine, which is a reaction product, was obtained. The results are shown in Table 2.

**Table 2**

| Lipase | Amount of recovery of raw material | Amount of reaction product |
|---|---|---|
| Nobozyme 435 | n.d. | 95.2% |
| LIPASE-QL | n.d. | 97.8% |

From the above results, it is found that Novozym 435 (Candida antarctica lipase B, manufactured by Novo Nordisk) and LIPASE-QL (Alcaligenes triacylglycerol lipase, manufactured by Meito Sangyo. Co.) consume all the raw material compound and produce only the compound in which the hydroxyl at the 5'-position has been levulinylated.

### Example 17

### Adenylyl-[3'→5']-adenylyl-[3'→5']-uridylyl-[3'→5']-guanylyl-[3'→5']-guanylyl-[3'→5']-adenylyl-[3'→5']-uridylyl-[3'→5']-guanylyl-[3'→5']-uridylyl-[3'→5']-adenylyl-[3'→5']-cytidylyl-[3'→5']-uridylyl-[3'→5']-uridylyl-[3'→5']-cytidylyl-[3'→5']-adenylyl-[3'→5']-uridylyl-[3'→5']-cytidylyl-[3'→5']-adenylyl-[3'→5']-cytidylyl-[3'→5']-thymidylyl-[3'→5']-thymidine

### Step 1: Synthesis of dimer blocks

By using the same method as in the step 1 of Example 12, 8.24 g of 5'-O-levulinyl-2'-O-(2-tetrahydrofuranyl)-N⁴-(4-anisoyl)adenosine 3'-(2-chlorophenoxy)phosphoryl 2'-0-(2-tetrahydrofuranyl)uridine 3'-[O-(2-cyanoethyl)(2-chlorophenyl)phosphate] (MS (EI) m/z = 1321.2 [M+Na]), 6.88 g of 5'-O-levulinyl-2'-O-(2-tetrahydrofuranyl)-N2-isobutyrylguanosine 3'-(2-chlorophenoxy)phosphoryl 2'-O-(2-tetrahydrofuranyl)-N²-isobutyrylguanosine 3'-[O-(2-cyanoethyl)(2-chlorophenyl)phosphate] (MS (EI) m/z = 1382.4 [M+Na]), 3.60 g of 5'-O-levulinyl-2'-O-(2-tetrahydrofuranyl)-N²-isobutyrylguanosine 3'-(2-chlorophenoxy)phosphoryl 2'-O-(2-tetrahydrofuranyl)uridine 3'-[O-(2-cyanoethyl)(2-chlorophenyl)phosphate] (MS (EI) m/z = 1273.4 [M+Na]), 16.12 g of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl)phosphate] (MS (EI) m/z = 1454.5 [M+Na]), 5.10 g of 5'-O-levulinyl-2'-O-(2-tetrahydrofuranyl)uridine 3'-(2-chlorophenoxy)phosphoryl 2'-O-(2-tetrahydrofuranyl)uridine 3'-[O-(2-cyanoethyl)(2-chlorophenyl)phosphate] (MS (EI) m/z = 1164.3 [M+Na]), 8.30 g of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-[O-(2-cyanoethyl)(2-chlorophenyl)phosphate] (MS (EI) m/z = 1454.5 [M+Na]), and 6.00 g of 5'-O-levulinyl-2'-O-(2-tetrahydrofuranyl)uridine 3'-(2-chlorophenoxy)phosphoryl 2'-O-(2-tetrahydrofuranyl)-N⁴-(4-anisoyl)cytidine 3'-[O-(2-cyanoethyl) (2-chlorophenyl)phosphate] (MS (EI) m/z = 1297.4 [M+Na]) were synthesized, respectively.
Further, 3.31 g of thymidine 3'-(2-chlorophenoxy)phosphoryl thymidine 3'-acetate, which is the compound or Reference example 3, was synthesized.

### Step 2: Synthesis of tetramer blocks

By using the dimer blocks synthesized in the step 1 of Example 17, and by the same method as in the step 2 of Example 12, 6.49 g of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate (MS (EI) m/z = 2062.0 [M+H]) was synthesized, and in the same manner as in the step 4 of Example 12, 4.20 g of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N²-isobutyrylguanosine 3'-(2-chlorophenoxy)phosphoryl 2'-O-(2-tetrahydrofuranyl)-N2-isobutyrylguanosine 3'-[O-(2-cyanoethyl)(2-chlorophenyl)phosphate] (MS (EI) m/z = 2492.7 [M+H]), 2.30 g of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N²-isobutyrylguanosine 3'-(2-chlorophenoxy)phosphoryl 2'-0-(2-tetrahydrofuranyl)uridine 3'-[O-(2-cyanoethyl)(2-chlorophenyl)phosphate] (MS (EI) m/z = 2383.3 [M+H]), 7.00 g of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)uridine 3'-(2-chlorophenoxy)phosphoryl 2'-0-(2-tetrahydrofuranyl)uridine 3'-[0-(2-cyanoethyl)(2-chlorophenyl)phosphate] (MS (EI) m/z = 2429.2 [M+Na]), and 8.38 g of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)cytidine 3'-[O-(2-cyanoethyl)(2-chlorophenyl) phosphate] (MS (EI) m/z = 2562.1 [M+Na]) were synthesized.

### Step 3: Synthesis of 5'-O-(4,4-dimethoxytrityl)-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N²-isobutyrylguanosine 3'-(2-chlorophenoxy)phosphoryl 2'-O-(2-tetrahydrofuranyl)-N²⁻isobutyrylguanosine 3'-[O-(2-cyanoethyl)(2-chlorophenyl)phosphate]

By using the compound synthesized in the step 2 of Example 17 and the compound of Example 10, 3.70 g of the above-mentioned compound was synthesized using the same method as in (B) of Example 11.

### Step 4: Synthesis of 5'-O-levulinyl-2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate

By using the compound synthesized in the step 2 of Example 17 and 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl thymidine 3'-(2-chlorophenoxy)phosphoryl thymidine 3'-acetate, 6.04 g of the above-mentioned compound was synthesized using the same methods as in the step 5 and the step 6 of Example 12.

### Step 5: Synthesis of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anysoyl)cytidine 3'-(2-chlorophenoxy)phosphoryl 2'-O-(2-tetrahydrofuranyl)-N ⁴-(4-anysoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anysoyl)cytidine 3'-(2-chlorophenoxy)phosphoryl 2'-O-(2-tetrahydrofuranyl)-N⁴-(4-anysoyl)adenosine 3'-(2-chlorophenoxy)phosphoryl 2'-O-(2-tetrahydrofuranyl)-N⁴-(4-anysoyl)cytidine 3'-(2-chlorophenoxy)phosphoryl thymidine 3'-(2-chlorophenoxy)phosphoryl thymidine 3'-acetate

By using the compound synthesized in the step 2 of Example 17 and the compound synthesized in the step 4 of Example 17, 7.04 g of the above-mentioned compound was synthesized using the same methods as in the step 5 and the step 6 of Example 12.

### Step 6: Synthesis of 5'-O-levulinyl-2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N²-isobutyrylguanosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-(-2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)-N⁴ (4-anisoyl)cytidine 3'-(2-chlorophenoxy)phosphoryl thymidine 3'-(2-chlorophenoxy)phosphoryl thymidine 3'-acetate

By using the compound synthesized in the step 2 of Example 17 and the compound synthesized in the step 5 of Example 17, 3.60 g of the above-mentioned compound was synthesized using the same methods as in the step 5 and the step 6 of Example 12.

### Step 7: Synthesis of 5'-O-(4,4-dimethoxytrityl)-2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N²-isobutyrylguanosine 3'-(2-chlorophenoxy)phosphoryl 2'-O-(2-tetrahydrofuranyl)-N²⁻isobutyrylguanosine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N²-isobutyrylguanosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)cytidine 3'-(2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)uridine 3'-(2-chlorophenoxy)phosphoryl 2'-O-(2-tetrahydrofuranyl)-N⁴-(4-anisoyl)cytidine 3'- (2-chlorophenoxy)phosphoryl 2'-O-(2-tetrahydrofuranyl)-N⁴-(4-anisoyl)adenosine 3'-(2-chlorophenoxy)phosphoryl 2'-O-(2-tetrahydrofuranyl)-N⁴-(4-anisoyl)cytidine 3' - (2-chlorophenoxy)phosphoryl thymidine 3'-(2-chlorophenoxy)phosphoryl thymidine 3'-acetate

By using the compound synthesized in the step 3 of Example 17 and the compound synthesized in the step 6 of Example 17, 4.27 g of the above-mentioned compound was synthesized using the same methods as in the step 12 and the step 13 of Example 12.

### Step 8: Deprotection and purification steps

To 100 mg of the compound synthesized in the step 7 of Example 17, 50% aqueous dioxane solution of 0.30 M pyridine-2-carboxaldoxime and N¹,N¹,N³,N³-tetramethyl guanidine was added, and the mixture was stirred at room temperature for 44 hours. The reaction solution was concentrated under reduced pressure, and the resulting substance was dissolved in 3 ml of pyridine. Then, 15 ml of 28% aqueous ammonia solution was added thereto, and the mixture was sealed and left at 55°C for 11 hours, and then left at room temperature for 12 hours. Further, the mixture was left at 55°C for 11 hours, and then left at room temperature for 12 hours. The reaction solution was concentrated, and to the residue, acetone was added. Then, the deposited precipitate was collected by centrifugation and washed 3 times with acetone, and then dried. The obtained powder was purified by reverse-phase ODS column chromatography (acetonitrile/0.05 M-TEAA buffer). To 22 mg of the resulting substance, 2 ml of 0.1 M sodium acetate/acetic acid buffer (pH 3.82) was added, and the mixture was left at 60°C for 1 hour. The reaction solution was desalted by reverse-phase ODS and lyophilized, and then purified by ion-exchange column chromatography (0 → 0.4 M NaCl/10 mM phosphate buffer). The resulting substance was desalted by reverse-phase ODS and lyophilized, whereby 12 mg of the objective compound, adenylyl-[3'→5']-adenylyl-[3'→5']-uridylyl-[3'→5']-guanylyl-[3'→5']-guanylyl-[3'→5']-adenylyl-[3'→5']-uridylyl-[3'→5']-guanylyl-[3'→5']-uridylyl-[3'→5']-adenylyl-[3'→5']-cytidylyl-[3'→5']-uridylyl-[3'→5']-uridylyl-[3'→5']-cytidylyl-[3'→5']-adenylyl-[3'→5']-uridylyl-[3'→5']-cytidylyl-[3'→5']-adenylyl-[3'→5']-cytidylyl-[3'→5']-thymidylyl-[3'→5']-thymidine, was obtained.
MALDI TOF MS: Calculated value: 6615.07, Measured value:
6615.34

### [Industrial Applicability]

The novel ribonucleic acid compound, which is the compound of the present invention, is a compound in which the hydroxyl at the 2'-position of ribose is protected with a protecting group that can be removed at 90% or more at a temperature in the range from 0°C to 60°C under acidic conditions at a pH value from 2 to 4 within 24 hours, the 3'-position of ribose is phosphotriesterified, and the 5'-position of ribose is levulinylated. The compound of the present invention is very useful for liquid-phase synthesis of oligo-RNA, and an important compound.
Further, a method of easily regioselectively levulinylating the hydroxyl at the 5'-position of ribose with a lipase for a ribonucleic acid compound in which the hydroxyl at the 2'-position of ribose is protected with a protecting group that can be removed at 90% or more at a temperature in the range from 0°C to 60°C under acidic conditions at a pH value from 2 to 4 within 24 hours, which is important for producing a phosphotriesterified ribonucleic acid compound, is provided.
Further, a novel liquid-phase synthesis method for oligo-RNA is provided.

## Claims

1. A ribonucleic acid compound represented by the following general formula (1): 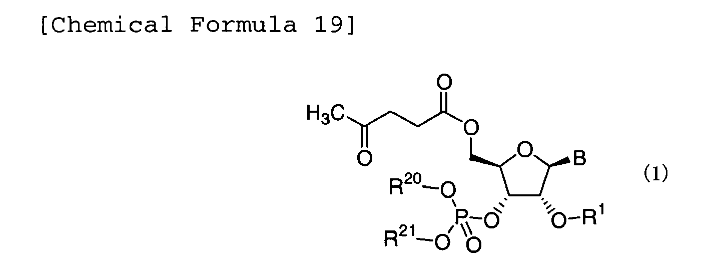 (wherein B represents adenine, guanine, cytosine or uracil or a modified form thereof; R¹ represents a protecting group which can be removed at 90% or more at a temperature in the range from 0°C to 60°C under acidic conditions at a pH value from 2 to 4 within 24 hours; R²⁰ represents H or an alkyl which may be substituted; and R²¹ represents an aryl which may be substituted or a monocyclic or bicyclic heterocyclic which may be substituted), or a salt thereof.

2. The ribonucleic acid compound or a salt thereof according to claim 1, wherein R¹ is 2-tetrahydrofuranyl or 1,3-dioxolan-2-yl.

3. The ribonucleic acid compound or a salt thereof according to claim 1 or 2, wherein R²⁰ is H, 2-cyanoethyl or 2,2,2-trichloroethyl, and R²¹ is 2-chlorophenyl or 2-chloro-4-tert-butylphenyl.

4. A method for producing a ribonucleic acid compound represented by the following general formula (3), comprising regioselectively levulinylating the hydroxyl at the 5'-position by allowing a levulinylating agent and a lipase to act on a ribonucleic acid compound represented by the following general formula (2): 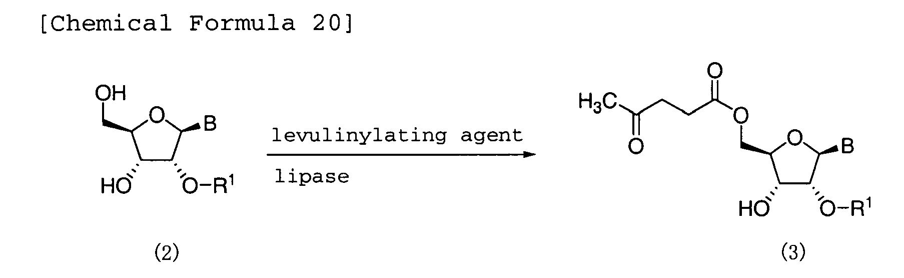 (wherein B represents adenine, guanine, cytosine or uracil or a modified form thereof; and R¹ represents a protecting group which can be removed at 90% or more at a temperature in the range from 0°C to 60°C under acidic conditions at a pH value from 2 to 4 within 24 hours).

5. A method for producing a ribonucleic acid compound (1) in which R²⁰ is H represented by the following general formula (1a) by allowing a phosphorylating reagent to act on a ribonucleic acid compound represented by the following general formula (3) produced by a production method including the step of regioselectively levulinylating the hydroxyl at the 5'-position by allowing a levulinylating agent and a lipase to act on a ribonucleic acid compound represented by the following general formula (2): 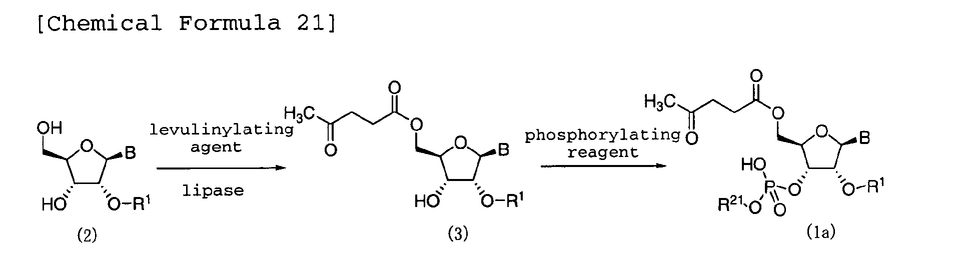 (wherein B represents adenine, guanine, cytosine or uracil or a modified form thereof; R¹ represents a protecting group which can be removed at 90% or more at a temperature in the range from 0°C to 60°C under acidic conditions at a pH value from 2 to 4 within 24 hours; and R²¹ represents an aryl which may be substituted or a monocyclic or bicyclic heterocyclic which may be substituted).

6. A method for producing a ribonucleic acid compound (1) in which R²⁰ is an alkyl which may be substituted represented by the following general formula (1b) by allowing a phosphorylating reagent and a reagent for protecting a phosphate group to act on a ribonucleic acid compound represented by the following general formula (3) produced by a production method including the step of regioselectively levulinylating the hydroxyl at the 5'-position by allowing a levulinylating agent and a lipase to act on a ribonucleic acid compound represented by the following general formula (2): 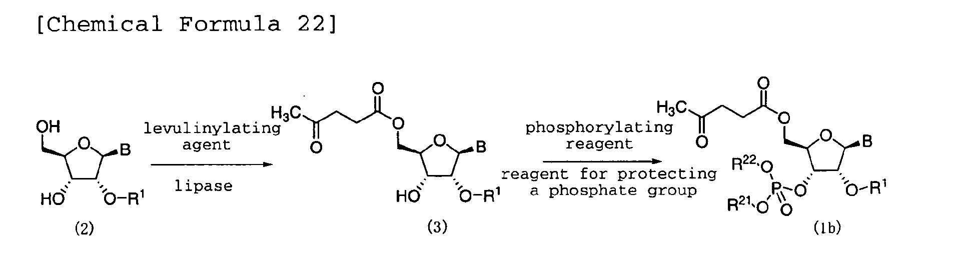 (wherein B represents adenine, guanine, cytosine or uracil or a modified form thereof; R¹ represents a protecting group which can be removed at 90% or more at a temperature in the range from 0°C to 60°C under acidic conditions at a pH value from 2 to 4 within 24 hours; R²¹ represents an aryl which may be substituted or a monocyclic or bicyclic heterocyclic which may be substituted; and R²² represents an alkyl which may be substituted).

7. The method for producing a ribonucleic acid compound according to any one of claims 4 to 6, wherein R¹ is 2-tetrahydrofuranyl or 1,3-dioxolan-2-yl.

8. The method for producing a ribonucleic acid compound according to any one of claims 4 to 7, wherein the levulinylating agent is levulinic acid, levulinic anhydride, a levulinate ester or a halide levulinate.

9. The method for producing a ribonucleic acid compound according to any one of claims 5 to 8, wherein the phosphorylating reagent is 2-chlorophenyl phosphoroditriazolide, 2-chlorophenyl-O,O-bis(1-benzotriazolyl)phosphate or 2-chloro-4-tert-butylphenyl phosphoroditriazolide.

10. The method for producing a ribonucleic acid compound according to any one of claims 6 to 9, wherein the reagent for protecting a phosphate group is 3-hydroxypropionitril or 2,2,2-trichloroethanol.

11. A liquid-phase synthesis method for an oligonucleotide compound represented by the following general formula (4): 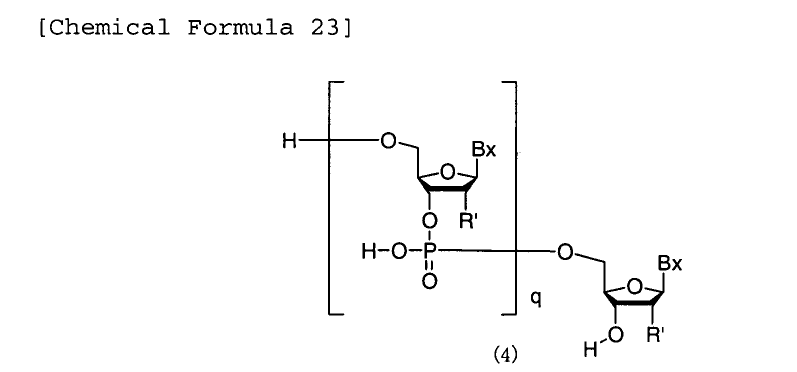 (wherein each Bx independently represents adenine, guanine, cytosine, uracil or thymine or a modified form thereof; q represents an integer in the range from 1 to 100; at least one of R' is hydroxyl and the others represent independently H or hydroxyl), comprising the following steps (a) to (f):
(a) producing a ribonucleic acid compound represented by the following general formula (3) by regioselectively levulinylating the hydroxyl at the 5'-position by allowing a levulinylating agent and a lipase to act on a ribonucleic acid compound represented by the following general formula (2): 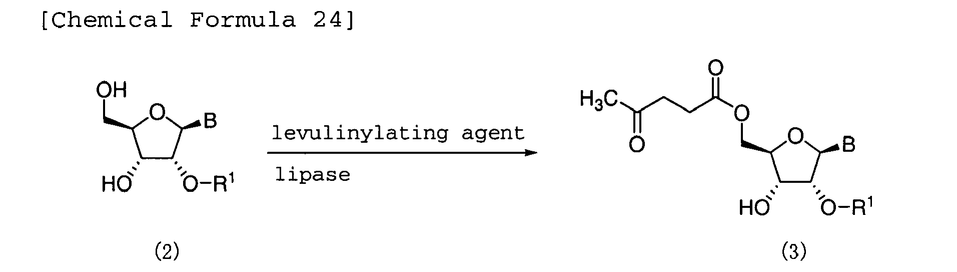 (wherein B represents adenine, guanine, cytosine or uracil or a modified form thereof; and R¹ represents a protecting group which can be removed at 90% or more at a temperature in the range from 0°C to 60°C under acidic conditions at a pH value from 2 to 4 within 24 hours);
(b) producing a ribonucleic acid compound represented by the following general formula (1a) by phosphorylating the hydroxyl at the 3'-position by allowing a phosphorylating reagent to act on a ribonucleic acid compound (3) produced by the step (a): 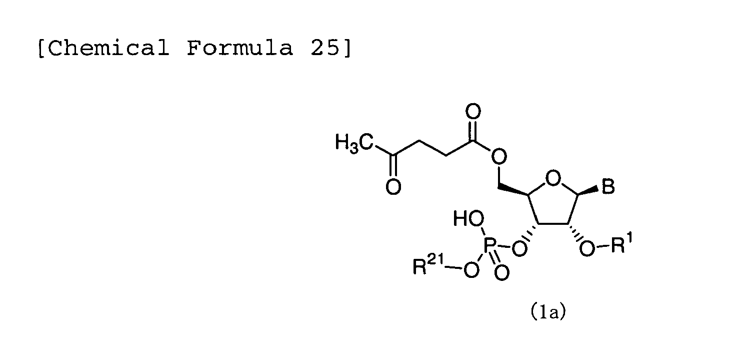 (wherein B and R¹ are the same as defined above; and R²¹ represents aryl which may be substituted or a monocyclic or bicyclic heterocyclic group which may be substituted);
(c) producing, separately from the step (b), a ribonucleic acid compound represented by the following general formula (1b) by allowing a phosphorylating reagent and a reagent for protecting a phosphate group to act on a ribonucleic acid compound (3) produced by the step (a): 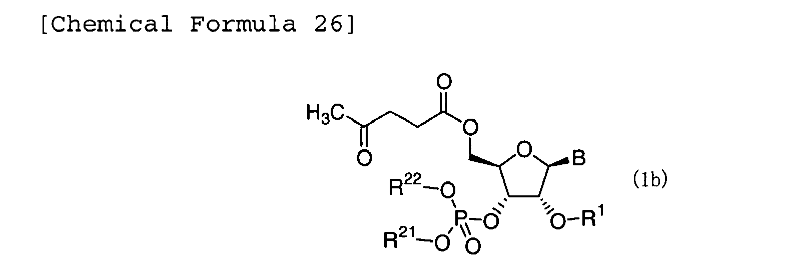 (wherein B, R¹ and R²¹ are the same as defined above; and R²² represents alkyl which may be substituted);
(d) producing a ribonucleic acid compound represented by the following general formula (5) by deprotecting levulinyl of the ribonucleic acid compound (1b) produced by the step (c): 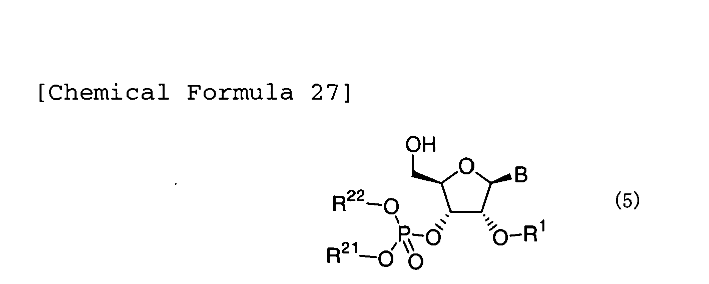 (wherein B, R¹, R²¹ and R²² are the same as defined above);
(e) producing an oligonucleotide compound represented by the following general formula (6) by stepwise oligomerization using as a monomer component, at least one of the ribonucleic acid compounds (1a) and (5) produced by the steps (b) and (d), respectively: 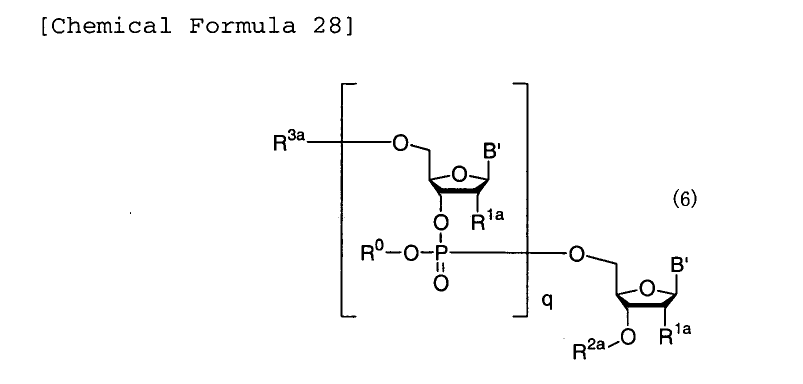 (wherein each B' independently represents adenine, guanine, cytosine, uracil or thymine or a modified form thereof; each R⁰ independently represents H, aryl which may be substituted or a monocyclic or bicyclic heterocyclic group which may be substituted; R^{3a} represents H, levulinyl or 4,4'-dimethoxytrityl; q is the same as defined above; at least one of R^{1a} is hydroxyl substituted with a protecting group which can be removed at 90% or more at a temperature in the range from 0°C to 60°C under acidic conditions at a pH value from 2 to 4 within 24 hours, and the others independently represent H or hydroxyl substituted with a protecting group which can be removed at 90% or more at a temperature in the range from 0°C to 60°C under acidic conditions at a pH value from 2 to 4 within 24 hours; and R^{2a} represents acyl or a phosphate group represented by the following general formula (7): 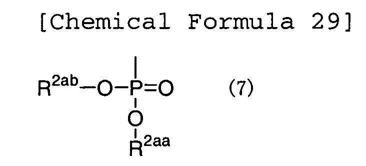 (wherein R^{2aa} represents aryl which may be substituted or a monocyclic or bicyclic heterocyclic group which may be substituted; and R^{2ab} represents H or alkyl which may be substituted); and
(f) deprotecting all the protecting groups of the oligonucleotide compound (6) produced by the step (e).

12. The liquid-phase synthesis method for an oligonucleotide compound according to claim 11, wherein R¹ is 2-tetrahydrofuranyl or 1,3-dioxolan-2-yl.

13. The liquid-phase synthesis method for an oligonucleotide compound according to claim 11 or 12, wherein q is an integer in the range from 1 to 100.

14. The liquid-phase synthesis method for an oligonucleotide compound according to any one of claims 11 to 13, wherein q is an integer in the range from 10 to 50.

15. The liquid-phase synthesis method for an oligonucleotide compound according to any one of claims 11 to 14, wherein the levulinylating agent is levulinic acid, levulinic anhydride, a levulinate ester or a halide levulinate.

16. The liquid-phase synthesis method for an oligonucleotide compound according to any one of claims 11 to 15, wherein the phosphorylating reagent is 2-chlorophenyl phosphoroditriazolide, 2-chlorophenyl-O,O-bis(1-benzotriazolyl)phosphate or 2-chloro-4-tert-butylphenyl phosphoroditriazolide.

17. The liquid-phase synthesis method for an oligonucleotide compound according to any one of claims 11 to 16, wherein the reagent for protecting a phosphate group is 3-hydroxypropionitril or 2,2,2-trichloroethanol.
